# EUROPEAN PATENT SPECIFICATION

(11) **EP 1 356 298 B1**
(45) Date of publication and mention of the grant of the patent: **18.05.2011**
(21) Application number: 01998835.1
(22) Date of filing: 30.11.2001
(51) Int. Cl.: G01N 33/68, C12Q 1/68, A61K 38/00, A61K 39/00

(54) **DIAGNOSIS OF DISEASE**
KRANKHEITSDIAGNOSE
DIAGNOSTIC DE MALADIE

(30) Priority: 30.11.2000 GB 0029225; 07.12.2000 GB 0029879
(43) Date of publication of application: 29.10.2003
(60) Divisional application: 10186268.8
(73) Proprietor: Crawford Healthcare Holdings Limited, Goostrey Cheshire CW4 8JP (GB)
(72) Inventor: TAZI-AHNINI, Rachid, Sheffield S10 2RX (GB); BAVIK,Claes, Sheffield S10 2RX (GB); WARD, Simon, Sheffield S10 2RX (GB); DUFF,Gordon, Sheffield S10 2RX (GB); CORK, Michael, Sheffield S10 2RX (GB)
(74) Representative: Finnie, Isobel Lara
(86) International application number: PCT/GB2001/005303
(87) International publication number: WO 2002/044736

(56) References cited:
- WO-A-00/58506
- WO-A-99/57149
- LOOMIS C A ET AL: "CHARACTERIZATION OF A KERATINOCYTE-SPECIFIC EXTRACELLULAR EPITOPE OF DESMOGLEIN IMPLICATIONS FOR DESMOGLEIN HETEROGENEITY AND FUNCTION" JOURNAL OF BIOLOGICAL CHEMISTRY, AMERICAN SOCIETY OF BIOLOGICAL CHEMISTS, BALTIMORE, MD, US, vol. 267, no. 23, 15 August 1992 (1992-08-15), pages 16676-16684, XP008038558 ISSN: 0021-9258
- OLAGUE-ALCALA M ET AL: "PEMPHIGUS FOLIACEUS SERA RECOGNIZE AN N-TERMINAL FRAGMENT OF BOVINE DESMOGLEIN 1" JOURNAL OF INVESTIGATIVE DERMATOLOGY, NEW YORK, NY, US, vol. 102, no. 6, 1994, pages 882-885, XP008038570 ISSN: 0022-202X
- WHITTOCK N V ET AL: "GENOMIC ORGANIZATION AND AMPLIFICATION OF THE HUMAN DESMOSOMAL CADHERIN GENES DSC1 AND DSC3, ENCODING DESMOCOLLIN TYPES 1 AND 3" BIOCHEMICAL AND BIOPHYSICAL RESEARCH COMMUNICATIONS, ACADEMIC PRESS INC. ORLANDO, FL, US, vol. 276, no. 2, 24 September 2000 (2000-09-24), pages 454-460, XP008038548 ISSN: 0006-291X
- HIRAKI A ET AL: "IMMUNOHISTOCHEMICAL STAINING OF DESMOSOMAL COMPONENTS IN ORAL SQUAMOUS CELL CARCINOMAS AND ITS ASSOCIATION WITH TUMOUR BEHAVIOUR" BRITISH JOURNAL OF CANCER, LONDON, GB, vol. 73, no. 12, 1996, pages 1491-1497, XP008038553 ISSN: 0007-0920
- MCGRATH J A: "HEREDITARY DISEASES OF DESMOSOMES" JOURNAL OF DERMATOLOGICAL SCIENCE, ELSEVIER SCIENCE PUBLISHERS, SHANNON, IR, vol. 20, no. 2, 1999, pages 85-91, XP008038547 ISSN: 0923-1811
- RICKMAN L ET AL: "N-TERMINAL DELETION IN A DESMOSOMAL CADHERIN CAUSES THE AUTOSOMAL DOMINANT SKIN DISEASE STRIATE PALMOPLANTAR KERATODERMA" HUMAN MOLECULAR GENETICS, OXFORD UNIVERSITY PRESS, SURREY, GB, vol. 8, no. 6, June 1999 (1999-06), pages 971-976, XP008038550 ISSN: 0964-6906
- ARMSTRONG D ET AL: "A NOVEL DESMOGLEIN 1 MUTATION IN STRIATE PALMOPLAMTAR KERATODERMA" JOURNAL OF INVESTIGATIVE DERMATOLOGY, NEW YORK, NY, US, vol. 114, no. 4, April 2000 (2000-04), page 826,ABSTRNO459, XP001202632 ISSN: 0022-202X
- BIERKAMP C ET AL: "DESMOSOMAL LOCALIZATION OF BETA-CATENIN IN THE SKIN OF PLAKOGLOBIN NULL-MUTANT MICE" DEVELOPMENT, COMPANY OF BIOLOGISTS, CAMBRIDGE,, GB, vol. 126, no. 2, January 1999 (1999-01), pages 371-381, XP008038528 ISSN: 0950-1991
- HUNT D M ET AL: "Clustered Cadherin Genes: A Sequence-Ready Contig for the Desmosomal Cadherin Locus on Human Chromosome 18" GENOMICS, ACADEMIC PRESS, SAN DIEGO, US, vol. 62, no. 3, 15 December 1999 (1999-12-15), pages 445-455, XP004444697 ISSN: 0888-7543
- JENISCH S ET AL: "Corneodesmosin gene polymorphism demonstrates strong linkage disequilibrium with HLA and association with psoriasis vulgaris." TISSUE ANTIGENS, vol. 54, no. 5, November 1999 (1999-11), pages 439-449, XP001149808 ISSN: 0001-2815
- RACHID TAZI AHNINI ET AL: "Novel genetic association between corneodesmosin (MHC S) gene and susceptibility to psoriasis" HUMAN MOLECULAR GENETICS, OXFORD UNIVERSITY PRESS, SURREY, GB, vol. 8, no. 6, June 1999 (1999-06), pages 1135-1140, XP002160633 ISSN: 0964-6906
- ISHIHARA M ET AL: "GENETIC POLYMORPHISMS IN THE KERATIN-LIKE S GENE WITHIN THE HUMAN MAJOR HISTOCOMPATIBILITY COMPLEX AND ASSOCIATION ANALYSIS ON THE SUSCEPTIBILITY TO PSORIASIS VULGARIS" TISSUE ANTIGENS, MUNKSGAARD, COPENHAGEN, DK, vol. 48, September 1996 (1996-09), pages 182-186, XP000982321 ISSN: 0001-2815 cited in the application
- HAFTEK M ET AL: "EXPRESSION OF CORNEODESMOSIN IN THE GRANULAR LAYER AND STRATUM CORNEUM OF NORMAL AND DISEASES EPIDERMIS" BRITISH JOURNAL OF DERMATOLOGY, XX, XX, vol. 137, no. 6, December 1997 (1997-12), pages 864-873, XP000853610 ISSN: 0007-0963
- KOCH P J ET AL: "TARGETED DISRUPTION OF THE PEMPHIGUS VULGARIS ANTIGEN (DESMOGLEIN 3) GENE IN MICE CAUSES LOSS OF KERATINOCYTE CELL ADHESION WITH A PHENOTYPE SIMILAR TO PEMPHIGUS VULGARIS" THE JOURNAL OF CELL BIOLOGY, ROCKEFELLER UNIVERSITY PRESS, US, vol. 137, no. 5, 2 June 1997 (1997-06-02), pages 1091-1102, XP002946611 ISSN: 0021-9525
- SIMON M ET AL: "Characterization and purification of human corneodesmosin, an epidermal basic glycoprotein associated with corneocyte-specific modified desmosomes" JOURNAL OF BIOLOGICAL CHEMISTRY. (MICROFILMS), AMERICAN SOCIETY OF BIOLOGICAL CHEMISTS, BALTIMORE, MD, US, XP002075788
- SIMON M ET AL: "Refined characterization of corneodesmosin proteolysis during terminal differentiation of human epidermis and its relationship to desquamation." THE JOURNAL OF BIOLOGICAL CHEMISTRY. UNITED STATES 8 JUN 2001, vol. 276, no. 23, 8 June 2001 (2001-06-08), pages 20292-20299, XP001149810 ISSN: 0021-9258
- SIMON MICHEL ET AL: "Persistence of both peripheral and non-peripheral corneodesmosomes in the upper stratum corneum of winter xerosis skin versus only peripheral in normal skin." JOURNAL OF INVESTIGATIVE DERMATOLOGY, vol. 116, no. 1, January 2001 (2001-01), pages 23-30, XP001149809 ISSN: 0022-202X
- ANONYMOUS: 'Accessing records in NCBI's sequence databases', [Online] Retrieved from the Internet: <URL:http://www.ornl.gov/sci/techresources/ Human_Genome/posters/chromosome/sequence.sh tml> [retrieved on 2008-06-20]

## Description

### FIELD

This invention relates to the diagnosis of diseases associated with reduced cell-cell adhesion. More particularly, the invention relates to the diagnosis of certain epidermal or skin diseases.

### BACKGROUND

The skin is a barrier that retains water within the body and prevents the penetration of environmental agents into the body. The barrier function of the skin is therefore essential to the maintenance of the internal homeostasis (Cork 1997). The epidermis is composed of layers of closely packed keratinocytes that are formed by division in the stratum basale (or germinative layer). As the keratinocytes move up through the prickle and granular layers, they differentiate and a rigid internal structure of keratin, microfilaments and microtubules is formed. The stratum corneum (horny layer) is composed of layers of flattened dead cells that have lost their nucleus, between which is a complex mixture of lipid and proteins.

The epidermal barrier is located in the stratum corneum and is dependent on strong adhesion between corneocytes (Egelrud, 2000). This adhesion is mediated by corneodesmosomes (Menton and Elisen, 1971 Chapman and Walsh, 1990; North et al, 1999). Corneodesmosin is a glycoprotein of comeodesmosomes.

The other component of the epidermal barrier is the lamellar lipids. The lipid is secreted into lamellar bodies in the stratum granulosum (Elias 1983). At the interface between the stratum granulosum and stratum corneum, the lipids are extracted from the granular cells into the intercorneoctye space. The lipids are then organised into highly organised multimellar bilayer structures (Landmann 1988). The stratum corneum can be visualised rather like a brick wall with the corneocytes forming the bricks and the lamellar lipids the mortar (Elias 1983). Corneocytes contain a water retaining substance, natural moisturising factor (NMF), which retains water within them. The high water content causes the corneocytes to swell, preventing the formation of fissures and cracks between them. The pliability and elasticity of the skin is directly related to its water content. Normal healthy stratum corneum has comparatively high water content.

Thus, the stratum corneum is a barrier that is continually being replaced by proliferation and differentiation of keratinocytes in the viable epidermis. In order to maintain a constant stratum corneum thickness at a given body site superficial parts of the stratum corneum must be continuously shed in the process of desquamation at a rate that balances their production. Impaired desquamation of corneocytes is characteristic of a number of diseases such as psoriasis, acne vulgaris, ichiosis and keratinosis pilaris, among others. In psoriasis, impaired desquamation of corneocytes causes an increased thickness of the stratum corneum and the barrier is usually enhanced.

Haftek, M et al, (1997) used immunohistochemical and immuno-ultrastructural investigations on corneodesmosin expression in various skin lesions characterised by abnormal production and/or of the retention of the horny layer of the epidermis to study the expression of corneodesmosin in the granular layer and stratum corneum of normal and diseased epidermis.

Simon, M et al, (2001) J Invest Dermatol No. 116:23-30 investigate the persistence of both peripheral and non-peripheral corneodesmosomes in the upper stratum corneum of winter xerosis skin versus only peripheral, in normal skin.

### SUMMARY

We demonstrate that proteolysis of corneodesmosomal proteins is a key process involved in desquamation.

Accordingly, we have discovered that increased, impaired or otherwise abnormal proteolysis of corneodesmosomal proteins is involved in several skin disorders. In particular, we have found out that increased proteolysis of adhesion proteins is involved in diseases characterised by impaired barrier function, and decreased proteolysis of adhesion proteins is involved in diseases characterised by impaired desquamation of corneocytes. We further demonstrate that mutations in genes encoding adhesion proteins result in reduced adhesion between epithelial cells.

Independent claims 1 and 5 define the invention in its broadest scope:
Specifically this invention provides a method of providing indications useful in the diagnosis of a disease, or susceptibility to a disease associated with decreased cell-cell adhesion between epithelial cells
wherein the disease is selected from the group consisting of atopic eczema, sebarrhoeic eczema, irritant contact dermatitis, allergic contact dermatitis, lung atopic asthma, post viral asthma, bronchial hyper-reactivity, chronic obstruction pulmonary disease, Crohn's disease, ulcerative colitis, coeliac disease, peptic ulceration, impetigo, viral warts, Molluscum Contagiosum, bacterial meningitis, viral meningitis, peptic ulceration associated with penetration of *Helicobacteria pylori,* skin melanoma, squamous cell carcinoma, basal cell carcinoma, cutaneous lymphoma, a skin cancer, a malignancy of gastrointestinal tract and a malignancy of the lung,
the method comprising detection of a mutation in corneodesmosin selected from detecting any one or more of:
(a) the presence of a T at position +1243 of a corneodesmosin nucleic acid;
(b) the absence of a H*ph*1 restriction enzyme site at position of+1243 of a corneodesmosin nucleic acid;
(c) the presence of a leucine (L) residue at position 394 of a corneodesmosin polypeptide (L20815);
(d) a mutation in a corneodesmosin nucleic acid which leads to (c);
(e) the following nucleotides or any one or more of the following amino acids at the relevant positions of a corneodesmosin nucleic acid or polypeptide:

| Nucleotide Position | 442 | 468 | 619 | 1215 | 1236 | 1243 | 1515 | 1593 |
|---|---|---|---|---|---|---|---|---|
| Nucleic acid(s) | A | AGT | | T A T | | T | G | T |
| Residue Position (1) | 127 | 137 | 186 | 385 | 392 | 394 | 485 | 511 |
| Residue Position (2) | 143 | 153 | 202 | 401 | 408 | 410 | 501 | 527 |
| Residue | D | S/- | F | S | S | L | D | D/N |

in which "Residue Position (1)" refers to the numbering of the sequence with accession number L20815, and "Residue Position (2)" refers to the numbering of the sequence accession number AF030130;
(f) the presence of a T at position 180 of a corneodesmosin nucleic acid;
(g) the presence of an F at position 40 of a corneodesmosin polypeptide having accession number L20815;
(h) the presence of an F at position 56 of a corneodesmosin polypeptide having accession number AF030130;
(i) a mutation in a corneodesmosin nucleic acid which leads to (g) or (h);
(j) the presence of a T at position 619 of a corneodesmosin nucleic acid;
(k) the presence of an F at position 186 of a corneodesmosin polypeptide; and
(l) a mutation in a corneodesmosin nucleic acid which leads to (k), in a sample of cells or bodily fluid obtained from a subject.

Specifically this invention also provdies a method of providing indications useful in the diagnosis of a disease or susceptibility to a disease associated with decreased cell-cell adhesion between epithelial cells in an individual,
wherein the disease is selected from the group consisting of: atopic eczema, sebarrhoeic eczema, irritant contact dermatitis, allergic contact dermatitis and impetigo,
the method comprising detecting the presence, absence or a modulated level of corneodesmosin or a fragment thereof, in a sample of cells or bodily fluid obtained from a subject.

### BRIEF DESCRIPTION OF THE FIGURES

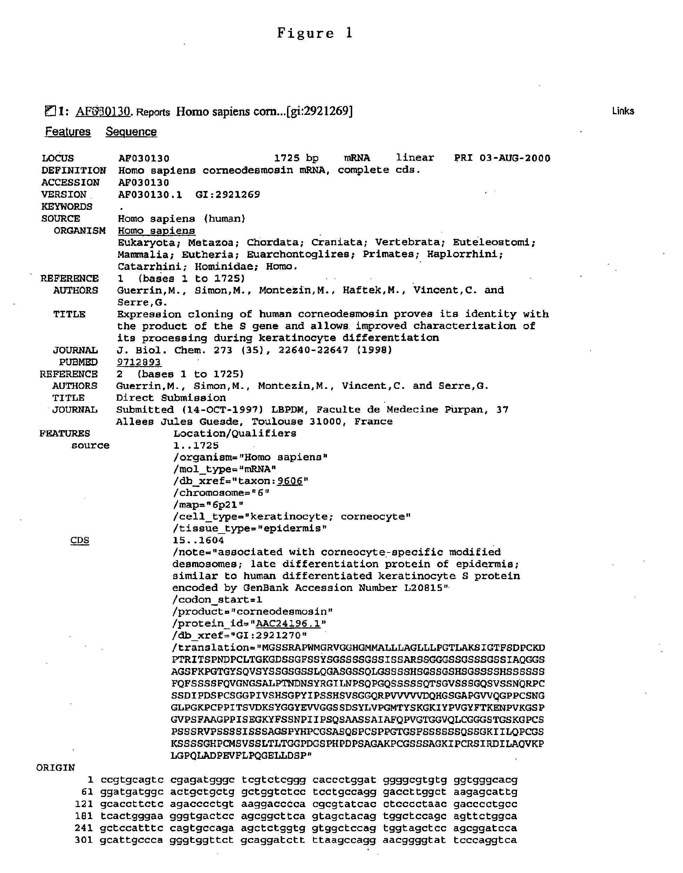

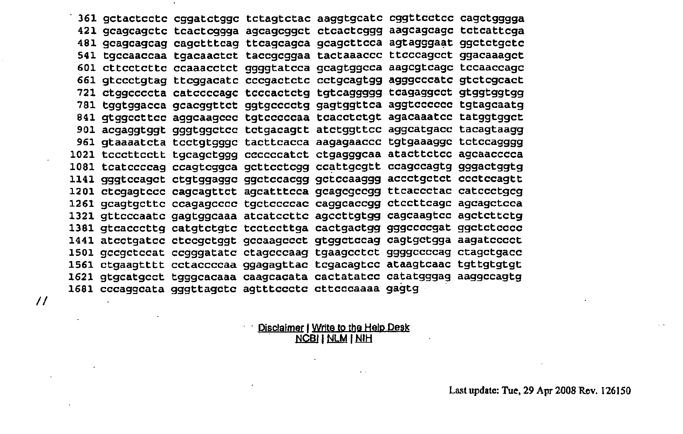

Figure 1 shows corneodesmosin sequence AF030130.1 from GenBank.

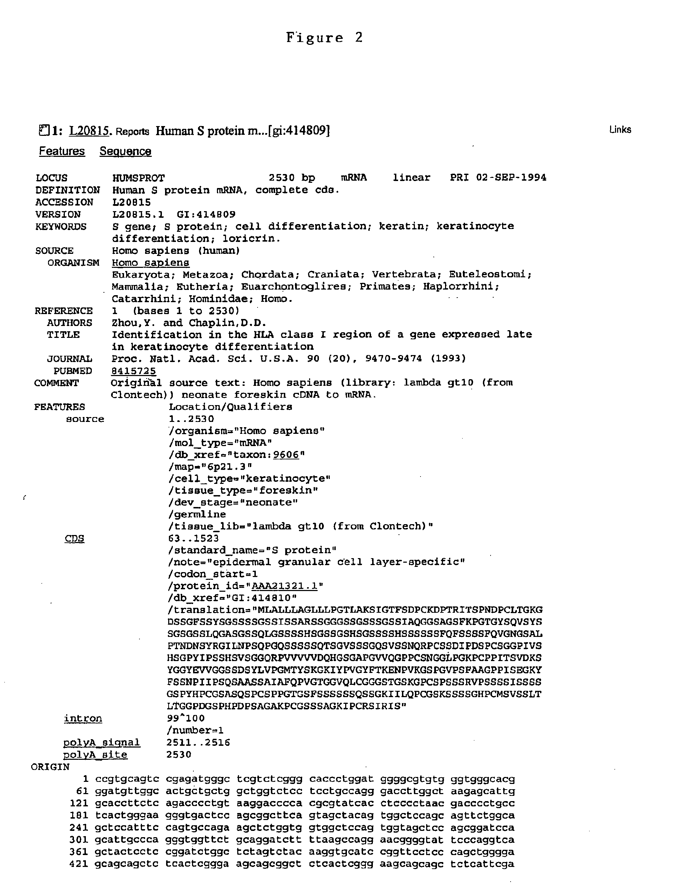

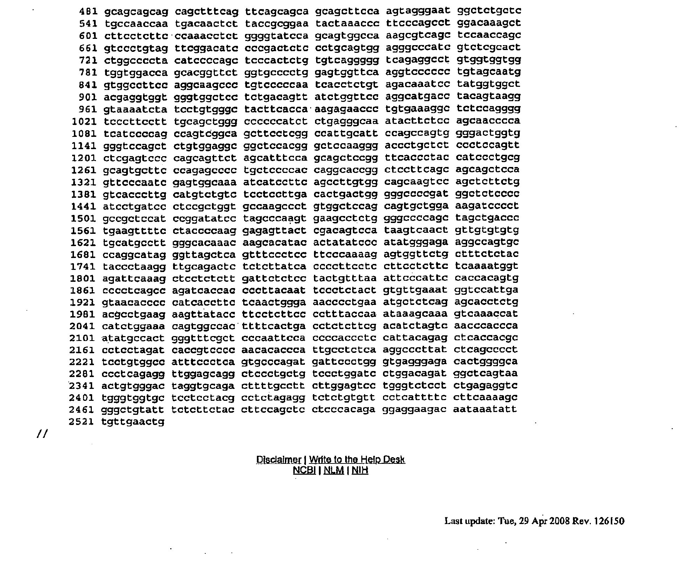

Figure 2 shows corneodesmosin sequence L20815.1 from GenBank.

### DETAILED DESCRIPTION

We demonstrate that regulated proteolysis of adhesion proteins is important for a healthy skin. We show that an underlying cause of various skin diseases is the breakdown in regulation of proteolysis of adhesion proteins, leading to an increased, decreased or otherwise abnormal adhesion between corneocytes. Such abnormal proteolysis may have various causes, including mutations in adhesion protein genes, mutations in proteases which act on adhesion proteins, and/or in protease inhibitors which act on such proteases and regulate their activity:

We show that treatment and prevention of such diseases may be achieved by modulating the proteolysis of adhesion proteins. We also demonstrate that detection of such mutations in adhesion proteins, proteases and/or protease inhibitors may be used to diagnose various skin diseases and/or susceptibility to such diseases.

Thus, for example, we show that mutations within genes encoding adhesion proteins, such as corneodesmosin (S gene) result in a reduced or increased adhesion between epithelial cells such as corneocytes, leading to disease. Furthermore, mutations within genes related to such adhesion proteins, for example, genes within the MHC epidermal gene cluster on chromosome 6p21 also result in reduced adhesion between epithelial cells. We identify specific mutations at various positions, including +1243, +180 and +619, and relate them to Group I and Group II diseases.

Furthermore, we show that mutations within genes encoding proteolytic enzymes (such as the stratum corneum chymotryptic enzyme (SCCE) and/or stratum corneum tryptic enzymes (SCTE) genes on chromosome 19 at the q13 band and related serine proteases genes in chromosome 17 result in an increased activity of these enzyme and as a result premature desquamation of corneocytes. In particular, we identify the association of an AACCAACC sequence with atopic eczema and other Group I diseases.

Furthermore, mutations in serine protease inhibitors genes such as SKALP and SLPI lead to failure of regulation of desquamation process. Thus, for example, mutations in the S, SCCE, SCTE, SKALP, SLPI genes or any combination of these genes result in impairment of the epidermal barrier function. Mutations in the genes occurring together increase the severity of the epidermal barrier function defect.

Accordingly, we provide for methods of diagnosis of a disease associated with abnormal cell-cell adhesion between epithelial cells by detecting such mutations. Furthermore, we also provide for methods of treatment of a disease associated with abnormal cell-cell adhesion between epithelial cells by regulating the expression of such protease and protease inhibitor genes.

Where reference is made to"diagnosis"of a disease, this should be taken to include both diagnosis of the disease itself, as well as susceptibility to the disease. The methods of diagnosis disclosed here may also be employed as methods of providing indications useful in the diagnosis of diseases.

Unless defined otherwise, all technical and scientific terms used herein have the same meaning as commonly understood by one of ordinary skill in the art (e. g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N. Y. and Ausubel et al., Short Protocols in Molecular Biology (1999) 4* Ed, John Wiley & Sons, Inc.. chemical methods, pharmaceutical formulations and delivery and treatment of patients.

### EPIDERMAL BARRIER FUNCTION

The methods and compositions described here are primarily concerned with the diagnosis and/or treatment of diseases associated with or characterised by abnormal epidermal barrier function.

The skin is a barrier that retains water within the body and prevents the penetration of environmental agents into the body. This ability to resist penetration is essential to the maintenance of the internal homeostasis (Cork 1997). The epidermis is composed of layers of closely packed keratinocytes that are formed by division in the stratum basale (or germinative layer). As the keratinocytes move up through the prickle and granular layers, they differentiate and a rigid internal structure of keratin, microfilaments and microtubules is formed. Within the epidermis, the barrier function is typically performed by the stratum corneum and is dependent on strong adhesion between corneocytes (Egelrud, 2000) mediated by corneodesmosomes (Menton and Elisen, 1971; Chapman and Walsh, 1990; North et al, 1999).

The stratum corneum (horny layer) is composed of layers of flattened dead cells that have lost their nucleus, between which is a complex mixture of lipid and proteins. The stratum corneum is a barrier that is continually being replaced by proliferation and differentiation of keratinocytes in the viable epidermis. In order to maintain a constant stratum corneum thickness at a given body site superficial parts of the stratum corneum must be continuously shed in the process of desquamation at a rate that balances their production.

The "barrier function" of the epidermis, or the "epidermal barrier function", as the terms are used in this document, is therefore intended to refer to the ability of an epidermal layer to resist the penetration of an external agent. Accordingly, the methods and compositions act by modulating the epidermal barrier function of an individual. In particular, the methods and compositions described here may be used to treat and/or diagnose diseases in which the epidermal barrier in individuals suffering from such diseases is modulated, i. e., enhanced or weakened, as compared to the epidermal barrier in normal individuals.

Measurement of barrier function may be done in various ways. For example, a Franz chamber and cadaver system (also known as a Franz chamber penetration system) may be used. This system measures barrier function by measuring the substances passing through it, and has the advantage in that it is a robust, quick and easy system.

### Impaired Barrier Function

Impaired barrier function is a characteristic of Group I diseases, described in detail below.

An epidermis has an impaired barrier function when it is more permeable to an external agent than a normal, healthy epidermis from the same or another individual. Thus, an epidermis with impaired barrier function allows more penetration of an external agent than otherwise. Preferably, an epidermis with impaired barrier function is 20%, 40%, 60%, 80% or 100% or more permeable to an external agent than a normal epidermis. Preferably, a molecule which is substantially unable to cross a normal epidermal barrier is able to cross the barrier of an epidermis with impaired barrier function.

Increase in penetration or permeability is preferably reflected by increase of mass of agent or drug, activity of an agent or drug (such as a relevant chemical, biological or enzymatic activity) which is capable of passing through the epidermal layer. Thus, an epidermis with impaired barrier function will therefore preferably enable penetration of 5%, 10%, 20%, 30%, 40%, 50%, 60%, 70%, 80% 90% or 100% or more agent or drug than a normal epidermis.

Increase in permeability or penetration may also be reflected by an increased ratio of molecules which pass through compared to those which are retarded, or alternatively, as compared to those which are applied. This ratio, N, may be increased by 5%, 10%, 20%, 30%, 40%, 50%, 60%, 70%, 80% 90% or 100% or more with the methods and compositions as described here.

Measurement of epidermal barrier function may be done in various ways. Similarly, the level or degree of penetration of an agent or composition can be determined by techniques known to those of skill in the art. For example, radiolabeling of an active compound or a tracer compound, followed by measurement of the amount of radiolabelled compound absorbed by the skin enables one of skill in the art to determine levels of the composition absorbed using any of several methods of determining skin penetration of the test compound. Measurement of the amount of radiolabelled compound which crosses the skin layer may also be made.

In a preferred embodiment, a Franz chamber and cadaver system (also known as a Franz chamber penetration system) is used to measure penetration of an agent. This system measures barrier function by allowing the measurement of amount of radiolabelled compound which passes through a piece of skin to a receptacle fluid. The Franz chamber has the advantage in that it is a robust, quick and easy system, and is described in more detail below.

### Enhanced Barrier Function

An epidermis which has increased barrier function is less permeable to an external agent than a normal, healthy epidermis from the same or other individual.

### GROUP I DISEASES: ASSOCIATED WITH DECREASED CELL-CELL ADHESION

In one embodiment, the methods disclosed here are suitable for treatment and diagnosis of diseases associated with decreased cell-cell adhesion, in particular of epithelial cells, in particular corneocytes. Such diseases are referred to in this document as"Group 1 diseases", and are therefore diseases of impaired or reduced barrier function.

Within the Group 1 diseases, three sub-groups may be identified. In each group the impaired barrier function is permitting the penetration of a xenobiotic through an epithelial barrier. Once the xenobiotic has penetrated through the epithelial barrier, it interacts with the host's cells to produce the disease phenotype.

A first subgroup of Group 1 diseases (Subgroup 1.1) is characterised by an impaired barrier that permits the penetration of an irritant, allergen or other xenobiotic. These xenobiotics interact with the body's immune system to produce an abnormal inflammatory response, which in turn causes tissue destruction. The immune response to the xenobiotic may be enhanced as the result of an associated genetic predisposition. Examples of such diseases affecting the skin include atopic eczema, sebarrhoeic eczema, irritant contact dermatitis, and allergic contact dermatitis. Examples of such diseass affecting the lung include: atopic asthma, post viral asthma/branchial hyper- reactivity, and chronic obstruction pulmonary disease. Examples of such diseases affecting the bowel include: Crohns disease, ulcerative colitis, coeliac disease, and peptic ulceration.

A second subgroup of Group 1 diseases (Subgroup 1.2) is characterised by an impaired barrier that permits the penetration of bacteria, virus, other micro-organisms or micro-organism products e. g. superantigenic exotoxin. The micro-organism and/or the micro-organism product then leads to a disease process. Examples of such diseases include atopic eczema, contact dermatits, impetigo, viral warts, Molluscum Contagiosum, meningitis (bacterial and viral), and peptic ulceration caused by or associated with penetration of Helicobacteria pylori.

A third subgroup of Group 1 diseases (Subgroup 1.3) is characterised by an impaired barrier that permits the penetration of a carcinogen. The carcinogen can thereby gain access to the epithelial stem cell population and can induce transformation. The carcinogen may act as a co-carcinogen with other environmental agents e. g. ultraviolet radiation. Examples of such diseases affecting the skin include melanoma, squamous cell carcinoma, basal cell carcinoma, cutaneous lymphoma, and other skin cancers. Examples of such diseases affecting the bowel include malignancies of the entire gastrointestinal tract. Lung malignancies are examples of diseases affecting the lung.

### Treatment of Group 1 Diseases

According to the methods and compositions described here, Group 1 diseases result from a decreased adhesion between epithelial cells, for example corneocytes in the skin. This can arise as a result of changes in the expression, activity and/or breakdown of adhesion proteins which modulate or are responsible for the adhesion between epithelial cells, for example, corneodesmosin, changes in the expression, activity and/or breakdown of proteases which break down the adhesion proteins, and/or changes in the expression, activity and/or breakdown of inhibitors of proteases which break down the adhesion proteins. We have discovered that a change in any, some or all of the above may result in decreased adhesion between epithelial cells such as corneocytes.

As an example, an adhesion protein whose structure is changed as a result of a genetic mutation may have reduced adhesion activity and/or be more vulnerable to degradation by proteolytic enzymes (proteases). Accordingly, therefore, treatment of Group 1 diseases may be affected by increasing the expression of an adhesion protein, for example, corneodesmosin. Alternatively or in conjunction treatment may be effected by enhancing the adhesion activity and/or protease resistance of the adhesion protein. Thus, the adhesion protein responsible for reduced cell-cell adhesion may be replaced or supplemented with another adhesion protein, or a variant of the first adhesion protein with enhanced adhesion activity and/or protease resistance.

Furthermore, an increase in the quantity, activity and/or bio-availability of proteases may cause increased break down of adhesion proteins. Accordingly, therefore, a Group 1 disease may be treated by reducing the expression and/or activity of a protease responsible for breaking down an adhesion protein. For example, the transcription and/or translation of such a protease may be down-regulated as a means to treat Group 1 diseases. Thus, the expression of proteases such SCCE and SCTE may be decreased at the transcriptional and/or translational level to treat a Group 1 disease.

Impaired barrier function as a result of reduced cell-cell adhesion may also happen where there is a decreased quantity, activity and/or bio-availability of an inhibitor of a protease which breaks down an adhesion protein. Thus, a combination of any, some or all of the above changes may result in a greater decrease in cell-cell adhesion and impaired barrier function.

Therefore, the expression and/or activity of a protease inhibitor which is capable of inhibiting a protease activity capable of breakdown of an adhesion protein may be up-regulated as a means to treat a Group 1 disease. The protease inhibitor whose expression and/or activity is up-regulated may be the natural protease inhibitor which physiologically inhibits the protease in question (i. e., the protease responsible for proteolysis of the adhesion protein). Such a protease inhibitor is referred to here as a "primary" protease inhibitor. Accordingly, we provide a method of treating a Group 1 disease by activating the transcription and/or translation of protease inhibitors (for example, SKALP and SLPI) to decrease the activity of proteases such as SCCE and SCTE. Furthermore, secondary protease inhibitors (i. e., protease inhibitors which are not normally involved in the physiological regulation of the protease activity) may also be used to supplement and/or replace the primary protease inhibitor activity.

Furthermore, the methods and compositions described here include the administration of a protease inhibitor or a fragment of a protease inhibitor to an individual suffering or likely to suffer from a Group I disease. The protease inhibitor and/or fragment may be administered in the form of a natural or synthethic peptide. Preferably, the peptide comprises an active portion of a protease inhibitor. Preferably, the peptide is capable of inhibiting one or more protease activities. Suitable peptides may be designed against any protease inhibitor, including Secretory Leukoprotease Inhibitor (SLPI), elafin protease inhibitor 3 (PI3 or SKALP) or cystatin A (CSTA).

Preferably, the peptide is selected from the group consisting of : CGKS (SB7a) and CGKS CVSPVKA (SB7b); KIIDGA; GDKIIDGA; GDKIID; KII; KIID; KIIDG; KIIDGA; LDPVD (651); KRDLK (652); LDPVDTPNP (653); LDPVDTPNPTRRKPG (654); CGKSCVSPVKA (644); CVSPVKA (643). In a preferred embodiment, the peptide comprises Peptide 643, Peptide 651 or Peptide 653.

Furthermore, where the natural inhibitors of proteases such as stratum corneum chymotrypsin/trypsin enzyme are found to be deficient or reduced in activity, these may be replaced or supplemented by secondary protease inhibitors such as chymotrypsin, soybean trypsin inhibitor, cathepsin G, or other protease inhibitors as known in the art. Primary protease inhibitors include antileukoprotease and elafin. Such primary and/or secondary protease inhibitors may be applied systemically, or preferably to the epithelial surface, in the form of a pharmaceutical composition as described in further detail below.

Using the skin as an example, a protease inhibitor may be applied to the \ epithelial surface, the protease inhibitor antagonising for example stratum corneum chymotrypsin enzyme. Such a protease inhibitor may be formulated in an emollient base. The protease inhibitor (s) will inhibit the degradation of adhesion proteins such as corneodesmosin and as a result increase the cohesion between the corneocytes and improve the structure and/or resistance of the epidermal barrier.

Expression of one or more genes coding for protease inhibitors, whether primary protease inhibitors or secondary protease inhibitors, may be used to increase protease inhibitor activity. For example, we provide for the subcutaneous (or otherwise) injection of expression vectors which express, for example, chymotrypsin, soybean trypsin inhibitor, cathepsin G, or other protease inhibitors in order to increase protease inhibitor activity to reduce proteolysis of the adhesion protein. Furthermore, as mentioned above, endogenous production of primary protease inhibitors may be enhanced by up-regulating transcription and/or translation of the relevant protease inhibitor genes, by means known in the art.

Furthermore, we provide for the deactivation of a pathogenic form of an epithelial or desmosomal protein, and/or the activation of non-pathogenic forms of epithelial or desmosomal proteins to treat Group 1 diseases. Thus, in heterozygote patients, the expression of a non-pathogenic form of a desmosomal protein, for example, corneodesmosin, may be up-regulated by means known in the art. Alternatively, or in conjunction, the expression of a pathogenic form of a desmosomal protein, for example, corneodesmosin, may be down-regulated by means known in the art. Pathogenic and non-pathogenic forms of such proteins may be identified by means known in the art such as genetic analysis and proteolysis profiles and as described in detail below.

### Diagnosis of Group 1 Diseases

Mutations in adhesion proteins, proteases and/or protease inhibitors are associated with Group I diseases. Such mutations are disclosed in Examples A1 to A6 and B 1 to B6. Any of these mutations may be detected in the relevant gene, nucleic acid or polypeptide in order to diagnose a Group I disease.

### GROUP II DISEASES: ASSOCIATED WITH INCREASED CELL-CELL ADHESION

Diseases characterised by increased cell-cell adhesion of epithelial cells, in particular corneocytes, are referred to in this document as "Group 2 diseases". Group 2 diseases are therefore diseases of increased or enhanced barrier function.

### Group II Diseases

Examples of Group 2 diseases include psoriasis, the ichthyoses, acne vulgaris and keratoses pilaris.

The Group 2 diseases may result from increased adhesion between epithelial cells, for example corneocytes in the skin. Increased adhesion results in an increased thickness of the stratum corneum. For example, in psoriasis and the ichthyoses, there is a generalised thickening of the stratum corneum. In acne there is a localised focal thickening of the stratum corneum at the entrance to the pilosebaceous duct.

Increased adhesion can arise as a result of changes in the expression, activity and/or breakdown of adhesion proteins which modulate or are responsible for the adhesion between epithelial cells, for example, corneodesmosin, changes in the expression, activity and/or breakdown of proteases which break down the adhesion proteins, and/or changes in the expression, activity and/or breakdown of inhibitors of proteases which break down the adhesion proteins. We have discovered that a change in any, some or all of the above may result in increased adhesion between epithelial cells such as corneocytes.

### ADHESION PROTEIN

Where the term "adhesion protein" is used in this document, this should be taken as reference to any protein, polypeptide or peptide which mediates adhesion between cells. Thus, an "adhesion protein" includes any protein which is involved in cell-cell adhesion.

Preferably, the adhesion protein mediates cell-cell interaction between epithelial cells, i.e., the adhesion protein is an epithelial cell adhesion protein. Preferably, the adhesion protein mediates cell-cell interaction between epidermal cells. More preferably, the adhesion protein mediates cell-cell adhesion between corneocytes. Preferably, the adhesion protein is located in a corneodesmosome. Most preferably, the adhesion protein is a desmosomal protein or a corneodesmosomal protein.

The adhesion protein may suitably be selected from the group consisting of adhesion proteins shown in Tables D 5.1. Preferably, the adhesion protein is selected from the group consisting of: corneodesmosin (AF030130), desoplakin (XM_004463), plakoglobin (NM_002230); (NM_021991), desmoglein 1 (XM-008810), desmocollin 1 (MX008687), envoplakin (XM008135 ; U72543), plectin 1 (NM000445), S100A2 (AI539439 ; M87068), keratin 6A (L42611), keratin 17 (Z19574),S1OOA8 (AI126134), S100A7 (AA586894), S10OA9), GB: W72424), SPRR2A), GB: M21302), SPRR1B (M19888), SPRK (AI923984), HCR (BAA81890), SEEK1 (BAA88130), SPR1 (BAB63315), STG (BAA88132), involucrin (NM005547), annexin A1/lipocortin (X05908), collagen, type VI, alpha 3 (COL6A3) (NM_004369), trichohyalin (NM_005547), and loricrin (XM_048902). GenBank accession numbers are provided in brackets.

Particularly preferred adhesion proteins comprise any of the desmosomal proteins corneodesmosin (also known as S; AF030130), desmoglein 1 (XM_008810), desmocollin 1 (MX_008687), desmoplakins I and II (XM_004463), plakoglobin (also known as PG; NM_002230) and plakophilin (also known as PP).

Highly preferred adhesion proteins include corneodesmosin, desmoglein 1, desmoglein 3, plakoglobin, desmoplakin, desmocollin 1, cnvoplakin, a proline-rich-protein, preferably a small proline-rich protein (SPRR), SPRR2A, SPRR1 B, SPRK, SPRR2E, SPRR2F, SPRR2B, SPRR2D, SPRR2C, SPRR2G, SPRR1 A, SPRR3, SPRR4, involucrin, or loricrin.

Mutations and polymorphisms in any of the above adhesion proteins which lead to or are associated with increased or decreased adhesion between epidermal cells may be detected by the methods described in detail in the Examples. Such changes may be detected as a means to identify or diagnose skin disease or susceptibility to such disease.

### Corneodesmosin

Mutations in adhesion proteins may be detected as a means to diagnose skin disease or susceptibility to such diseases. In a highly preferred embodiment, the adhesion protein is corneodesmosin.

The corneodesmosin gene is very polymorphic. To date there are nineteen variants described in the literature (Kasahara et al 1997, Jenisch et al, 1999). These polymorphisms are highly conserved rather than sporadic and we believe that they have been selected during vertebrate evolution. Because of the barrier function of the skin some forms of the corneodesmosin giving a strong resistance and/or dynamic function to the skin have been selected. A strong association has been shown between corneodesmosin variant at position +1243 and chronic plaque psoriasis (Tazi-Ahnini et al, 1999b). This association is even stronger in guttate psoriasis (Tazi-Ahnini et al, 1999b). The substitution at position +1243 gives amino acid change L394S. We believe that this substitution interferes with the processing of the corneodesmosin, thus contributing to the disruption of desquamation. We have found that nine of the corneodesmosin polymorphisms giving amino acid change (Ser143/Asp, Ser153/-, Leu180/Phe, Ser202/Phe, Ser401/Gly, Ser408/Ala, Gly409/Val, Ser410/Leu, Asp527/Asn) have an important function in keratinocyte maturation and desquamation process. The screening method for these polymorphisms is described by Jenisch et al, 1999 and Guerrin et al, 2001. We show that there is a strong relationship between proteolysis process of the corneodesmosin and the sensitivity of normal skin, and also with diseases including psoriasis, acne, eczema in which the barrier function is disturbed.

We show in the Examples that mutations in corneodesmosin are associated with skin diseases, particularly diseases of decreased adhesion.

In particular, we demonstrate in the Examples that a T nucleotide in position +1243 of the corneodesmosin sequence (AF030130) is associated with diseases of decreased skin adhesion. The presence of a T nucleotide at this position leads to a threonine (T) residue at position 394 of the encoded corneodesmosin polypeptide. Detection of either or both may be used to diagnosis disease.

We therefore disclose the diagnosis of a disease of decreased skin adhesion (a Group I disease) or susceptibility to such a disease in an individual, by detecting the presence of a T at position +1243 of a corneodesmosin nucleic acid in an individual. We also provide for the diagnosis of such a disease, or susceptibility to it, by detection of a threonine (T) residue at position 394 of a corneodesmosin polypeptide in an individual. Group I diseases may also be diagnosed by detection of a CD5 or CD6 corneodesmosin allele in an individual; the sequence of the CD5 and CD6 alleles is described in Jenisch et al. 1999.

Preferably, the Group I disease is eczema or dermatitis. More preferably, the Group I disease is atopic eczema or dermatitis hyperformis. Other examples of Group I diseases are set out in a separate section in this document.

Other mutations are disclosed in the Examples at +619 and +180 of corneodesmosin nucleic acid ; these and the corresponding polypeptide changes may be detected to diagnose a Group I or Group 11 disease. Other diagnosis and treatment methods employing corneodesmosin are disclosed in the Examples.

### PROTEASES

Proteases which may be used in the methods and compositions described here include the following: Apoptosis-related cysteine protease (CASP14) mRNA (NM_012114), Transglutaminase 1 (TGM1) (M98447), TGM2 (XM_009482), TGM4 (XM_056203), TGM5 (XM_007529), TGM7 (NM_052955), TGM3 (L10386), phospholipases A (2) (BC013384), CD47 antigen (X69398), Kallilkrein 8 (AB008390), AD024 protein (XM_002642), SCCE (XM_009002), Defensin beta2 (AF0711216), Interferon a inducible protein 27 (X67325), Fatty acid binding protein FABP5 (M94856), SCTE (XM_009000), kallikrein 1, renal/pancreas/salivary (KLK1) (XM_047300), Homo sapiens kallikrein 2, prostatic (KLK2) (XM_031757), kallikrein 3, (prostate specific antigen) (KLK3) (XM_031768), kallikrein 6 (neurosin, zyme) (KLK6) (XM_055658), kallikrein 4 (prostase, enamel matrix, prostate) (KLK4) (XM_008997), membrane-type serine protease 1 (AF133086), Human skin collagenase (M13509), collagenase MMP-1 (LOC116389), collagenase MMP-12 (U78045), collagenase MMP-9 (NM_004994), collagenase MMP-3 (U78045), collagenase MMP- 28 (AF219624), caspase 7 (BC015799), Caspase 5 (NM_004347), Caspase-14 (NM_012114), ubiquitin specific protease USP-5 (NM_003481), ubiquitin specific protease USP-11 (NM004651), ubiquitin specific protease USP 6 (NM_004505), ubiquitin specific protease USP 26 (NM_031907), ubiquitin specific protease (USP 28) (NM_020886), 26S protease subunit 4, LILRB 1 (AF004230), Signal trasducer and activator of transcription 1,91 kDa (STAT1) (977935), proteasome (prosome, macropain) subunit 6 (PSMA6) (X59417), TPS1 (NM_003293), TPSB1 (XM_016204), TPSG1 (XM_008123), protease nexin-II (XM_047793), Glia derived nexin precursor (P07093), 26S protease regulatory subunit S10B, and PCOLN3 (XM_047524).

Preferably, the protease is one which is involved in or is capable of proteolysis of an adhesion protein, preferably an epidermal cell-cell adhesion protein such as corneodesmosin. Accordingly, in a preferred embodiment, the protease comprises a protease selected from the group consisting of: Stratum Corneum Chymotryptic Enzyme (SCCE), Stratum Corneum Tryptic Enzyme (SCCE) kallikrein 1, kallikrein 2, kallikrein 3, kallikrein 4, kallikrein 6 and kallikrein 8.

### Stratum Corneum Chymotryptic Enzyme

### (SCCE) Stratum Corneum Tryptic Enzyme (SCTE)

Stratum corneum chymotryptic enzyme (SCCE) or kallikrein 7 (KLK7) as well as stratum corneum tryptic enzyme (SCTE) or kallikrein 5 (KLK5) are members of large serine protease family. Analysis of their expression profiles suggests their skin specificity. Both enzymes are expressed in high suprabasal keratinocytes and interfollicular epidermis and have maximum activity at the physiologic pH of stratum corneum (Ekholm et al, 2000; Egelrud, 1993, Ekholm and Egelrud, 1998).

SCCE and SCTE arc transported to the stratum corneum extracellular space during cornification. SCCE is produced as an inactive precursor and there is a need for an activating enzyme to confer to SCCE its proteolytic activity. SCTE is thought to be the enzyme involved in SCCE activation. A cluster of kallikrein genes including SCCE and SCTE has been mapped in chromosome 19q13. 3-13.4. This includes at least 6 structurally and evolutionary-related members (KLK1, KLK2, KLK3, KLK4, SCCE and SCTE). Each of these kallikrein genes may be used in the methods and compositions described here.

Of those SCCE and SCTE are mainly expressed in skin. They are also expressed in other tissues such as brain, kidney, mammary and salivary glands (Yousef et al, 2000; Yousef and Diamandis, 1999).

We show that the presence of AACCAACC in SCCE nucleic acid is associated with diseases of decreased skin adhesion, in particular eczema (preferably atopic eczema). Therefore, we provide for the diagnosis of a disease of decreased skin adhesion (a Group I disease) or susceptibility to such a disease in an individual, by detecting the presence of an AACC repeat or the sequence AACCAACC of a SCCE nucleic acid in an individual.

Homologues of SCCE and SCTE, in particular, homologues which are localised in the skin and/or act on adhesion proteins, may also be used in the methods of diagnosis and treatment described here. Such homologues may be identified by conventional library screening using an SCCE and/or SCTE probe, as well as database searching using relevant sequences.

Examples of other proteases useful in the methods and compositions described here include aminopeptidase M, carboxypeptidase P, carboxypeptidase Y, caspase 1,4,5, caspase 2,3,7, caspase 6,8,9, chymotrypsin, Factor Xa, pepsin, TEV, thrombin, trypsin etc.

### PROTEASE INHIBITORS

We demonstrate that regulation of desquamation is not only controlled by proteases (for example, SCCE and SCTE) but also by their inhibitors. Protease inhibitors which may be used in the methods and compositions described here include the following:

### Secretory Leukoprotease Inhibitor (SLPI) and Elafin

Several serine proteases are present in human epidermis including antileukoprotease (skin-derived antileukoprotease) and elafin. Antileukoprotease is a powerful inhibitor of SCCE. Elafin protease inhibitor 3 (PI3 or SKALP) is another serine proteases inhibitors produced by keratinocytes. It is over-expressed in the subcomeal layers of lesional psoriatic skin and in other skin disorders such as Behcet's syndrome, Sweet's syndrome, pyoderma gangrenosum and cutaneous allergic vasculitis (Tanaka et al. 2000). We have found that changes of SKALP expression affects SCCE and SCTE activities and hence disturbs the structure of superficial layers of the epidermis in skin disorders such as psoriasis and eczema.

The terms "Secretory Leukoprotease Inhibitor","SLPI" and "antileukoprotease" as used in this document are intended to be synonymous with each other.

Elafin is also known as skin-derived antileukoprotease, SKALP and protease inhibitor 3 (PI3). Accordingly, these terms are intended to be synonymous to each other, as used in this document.

### Cystatin A

The Examples demonstrate the presence of various polymorphisms in cystatin A, particularly in the promoter region of cystatin A. we therefore provide for the diagnosis of a disease, preferably a Group 1 and/or a Group 11 disease, by detecting the presence and/or absence of these polymorphism.

We show in the Examples that cystatin A is highly expressed in disease with increse adhesion (e. g. acne and psoriasis) and down-regulated in diseases with defective skin barrier (e. g. eczema). Accordingly, activity or level of cystatin A may be up-regulated as a means to treat a Group I diesease. Cystatin A level and/or activity may be down-regulated as a means to treat a Group II diesease.

We therefore disclose the diagnosis of a Group I disease or susceptibility to a Group I disease (preferably eczema or susceptibility to eczema, preferably atopic eczema) in an individual, by detecting modulation, preferably down-regulation of expression of cystatin A in an individual.

We further provide for the diagnosis of a Group II disease or susceptibility to a Group II disease (preferably eczema and/or psoriasis) in an individual, by detecting modulation, preferably up-regulation of expression of cystatin A in an individual.

We provide for the treatment or prevention of a Group I disease (preferably eczema or susceptibility to eczema, preferably atopic eczema) in an individual, by modulating, preferably up-regulating expression of cystatin A in an individual.

We further provide for the treatment or prevention of a Group II disease or susceptibility to a Group 11 disease (preferably eczema and/or psoriasis) in an individual, by modulating, preferably down-regulating expression of cystatin A in an individual.

### POLYPEPTIDES

The methods and compositions described here provide generally for a number of polypeptides, together with fragments, homologues, variants and derivatives thereof. Suitably useful polypeptides include adhesion proteins, proteases or protease inhibitors, or fragments, homologues, varianst or derivatives thereof.

Thus, we disclose variants, homologues or derivatives of an amino acid sequence of an adhesion proteins, protease or protease inhibitor, as well as variants, homologues or derivatives of a nucleotide sequence encoding such amino acid sequences. Each of these may be used for the diagnosis of a Group I disease.

The polypeptides, variants, homologues, fragments and derivatives disclosed here may be labelled with a revealing label. The revealing label may be any suitable label which allows the polypeptide, etc to be detected. Suitable labels include radioisotopes, e. g. ¹²⁵I, enzymes, antibodies, polynucleotides and linkers such as biotin. Labelled polypeptides may be used in diagnostic procedures such as immunoassays to determine the amount of a polypeptide in a sample. Polypeptides or labelled polypeptides may also be used in serological or cell-mediated immune assays for the detection of immune reactivity to said polypeptides in animals and humans using standard protocols.

A polypeptide, variant, homologue, fragment or derivative disclosed here, optionally labelled, my also be fixed to a solid phase, for example the surface of an immunoassay well or dipstick. Such labelled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

### FRAGMENTS

We also provide for nucleic acids and polypeptides or peptides which are fragments of the adhesion protein, protease or protease inhibitor nucleic acids and polypeptides disclosed here.

Preferably such nucleic acid and polypeptide fragments comprise, preferably consist of, 5,6,7,8,9,10,11,12,13,14,15,16,17,18,19,20,21,22,23,24,25,26,2728,29,30,31,32, 33, 34, 35, 36, 37, 38, 39, 40, 41, 42, 43, 44, 45, 46, 47, 48, 49, 50, 51, 52, 53, 4, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85,86,87,88,89,90, 91, 92, 93, 94, 95, 96, 97, 98, 99, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370,375, 380, 385, 390, 395, 400, 405, 410, 415, 420, 425, 430, 435, 440,445,450,455, 460, 465, 470, 475,480,485, 490, 495 or 500, or more residues from a relevant sequence (i.e., any of the sequences disclosed here). Preferably, such fragments comprise biological activity, preferably adhesion, protease or protease inhibitor activity.

A nucleic acid of use in the methods and compositions described here may comprise a viral or non-viral DNA or RNA vector, where non-viral vectors include, but are not limited to, plasmids, linear nucleic acid molecules, artificial chromosomes, condensed particles and episomal vectors. Expression of heterologous genes has been observed after injection of plasmid DNA into muscle (Wolff. *et al.,* 1990; Felgner *et al.,* 1996), thyroid (Sikes *et al.,* 1994), melanoma (Vile *et al.,* 1993), skin (Hengge *et al.,* 1995), liver (Hickman et *al.,* 1994) and after exposure of airway epithelium (Meyer *et al.,* 1995).

As used herein, the term "nucleic acid"is defined to encompass DNA and RNA or both synthetic and natural origin which DNA or RNA may contain modified or unmodified deoxy-or dideoxy-nucleotides or ribonucleotides or analogues thereof. The nucleic acid may exist as single-or double-stranded DNA or RNA, an RNA/DNA heteroduplex or an RNA/DNA copolymer, wherein the term"copolymer"refers to a single nucleic acid strand that comprises both ribonucleotides and deoxyribonucleotides.

The term "synthetic", as used herein, is defined as that which is produced by *in vitro* chemical or enzymatic synthesis.

### NUCLEIC ACIDS

The methods and compositions described here provide generally for a number of nucleic acids, together with fragments, homologues, variants and derivatives thereof. Suitably useful nucleic acids include those which encode adhesion proteins, proteases or protease inhibitors, or fragments, homologues, varianst or derivatives thereof.

As used here in this document, the terms "polynucleotide", "nucleotide", and nucleic acid are intended to be synonymous with each other. "Polynucleotide" generally refers to any polyribonucleotide or polydeoxribonucleotide, which may be unmodified RNA or DNA or modified RNA or DNA. "Polynucleotides" include, without limitation single-and double-stranded DNA, DNA that is a mixture of single- and double-stranded regions, single-and double-stranded RNA, and RNA that is mixture of single-and double-stranded regions, hybrid molecules comprising DNA and RNA that may be single-stranded or, more typically, double-stranded or a mixture of single-and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide"embraces chemically, enzymatically or metabolically modified forms of polynucleotides as typically found in nature, as well as the chemical forms of DNA and RNA characteristic of viruses and cells. "Polynucleotide"also embraces relatively short polynucleotides, often referred to as oligonucleotides.

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

We also provide nucleic acids which are fragments, homologues, variants or derivatives of the relevant nucleic acids (e. g., an adhesion protein, protease or protease inhibitor encoding nucleic acid).

### Variants, Derivatives and Homologues

Nucleic acid variants, fragments, derivatives and homologues may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3'and/or 5'ends of the molecule. For the purposes of this document, it is to be understood that the polynucleotides may be modified by any method available in the art. Such modifications may be carried out in order to enhance the in vivo activity or life span of polynucleotides of interest.

Where the polynucleotide is double-stranded, both strands of the duplex, either individually or in combination, are encompassed by the methods and compositions described here. Where the polynucleotide is single-stranded, it is to be understood that the complementary sequence of that polynucleotide is also included.

The terms "variant", "homologue" or "derivative"in relation to a nucleotide sequence include any substitution of, variation of; modification of, replacement of, deletion of or addition of one (or more) nucleic acid from or to the sequence. Preferably said variant, homologues or derivatives code for a polypeptide having biological activity. Preferably, such fragments, homologues, variants and derivatives of the adhesion proteins, proteases and protease inhibitors comprise modulated enzymatic activity, for example, adhesion protein activity (e. g., ability to enable the adhesion between two cells), protease activity or protease inhibitor activity. Thus, for example, we provide fragments, homologues, variants and derivatives of the relevant polypeptides which comprise a lower adhesion protein, protease or protease inhibitor activity compared to unmodified polypeptides. In particular, we provide fragments, homologues, variants and derivatives of adhesion proteins, which display reduced or enhanced adhesion activity. Fragments, homologues, variants and derivatives of proteases and protease inhibitors are also provided, which display reduced or enhanced protease or protease inhibitor activity.

Assays for adhesion activity, protease activity and protease inhibitor activity are known in the art.

As indicated above, with respect to sequence identity, a "homologue" has preferably at least 5% identity, at least 10% identity, at least 15% identity, at least 20% identity, at least 25% identity, at least 30% identity, at least 35% identity, at least 40% identity, at least 45% identity, at least 50% identity, at least 55% identity, at least 60% identity, at least 65% identity, at least 70% identity, at least 75% identity, at least 80% identity, at least 85% identity, at least 90% identity, or at least 95% identity to the relevant sequence shown in the sequence listings. Thus, in particular, we provide the use of homologues of adhesion proteins, proteases and protease inhibitors having such identity in the treatment of Group I and Group II diseases.

More preferably there is at least 95% identity, more preferably at least 96% identity, more preferably at least 97% identity, more preferably at least 98% identity, more preferably at least 99% identity. Nucleotide identity comparisons may be conducted as described above. A preferred sequence comparison program is the GCG Wisconsin Bestfit program described above. The default scoring matrix has a match value of 10 for each identical nucleotide and-9 for each mismatch. The default gap creation penalty is-50 and the default gap extension penalty is-3 for each nucleotide.

### Hybridisation

We further describe nucleotide sequences that are capable of hybridising selectively to any of the sequences presented herein, or any variant, fragment or derivative thereof, or to the complement of any of the above. Nucleotide sequences are preferably at least 15 nucleotides in length, more preferably at least 20, 30 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, may be at least 40% homologous, at least 45% homologous, at least 50% homologous, at least 55% homologous, at least 60% homologous, at least 65% homologous, at least 70% homologous, at least 75% homologous, at least 80% homologous, at least 85% homologous, at least 90% homologous, or at least 95% homologous to the corresponding nucleotide sequences presented herein. Preferably, such polynucleotides will be generally at least 70%, preferably at least 80 or 90% and more preferably at least 95% or 98% homologous to the corresponding nucleotide sequences presented herein over a region of at least 20, preferably at least 25 or 30, for instance at least 40,60 or 100 or more contiguous nucleotides.

The term "selectively hybridizable" means that the polynucleotide used as a probe is used under conditions where a target polynucleotide is found to hybridize to the probe at a level significantly above background. The background hybridization may occur because of other polynucleotides present, for example, in the cDNA or genomic DNA library being screening. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e. g. with ³²p.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

In a preferred aspect, we provide nucleotide sequences that can hybridise to the adhesion protein, protease or protease inhibitor nucleic acids, fragments, variants, homologues or derivatives under stringent conditions (e.g. 65°C and 0. 1xSSC {IxSSC = 0. 15 M NaCl, 0.015 M Na₃ Citrate pH 7.0).

### Generation of Homologues, Variants and Derivatives

Polynucleotides which are not 100% identical to the sequences of the present disclosure but which are also included, as well as homologues, variants and derivatives of the adhesion proteins, proteases and protease inhibitors can be obtained in a number of ways. Other variants of the sequences may be obtained for example by probing DNA libraries made from a range of individuals, for example individuals from different populations. For example, homologues may be identified from other individuals, or other species. Further recombinant nucleic acids and polypeptides may be produced by identifying corresponding positions in the homologues, and synthesising or producing the molecule as described elsewhere in this document.

In addition, other viral/bacterial, or cellular homologues of adhesion proteins, proteases and protease inhibitors, particularly cellular homologues found in mammalian cells (e. g. rat, mouse, bovine and primate cells), may be obtained and such homologues and fragments thereof in general will be capable of selectively hybridising to a human adhesion protein, protease or protease inhibitor. Such homologues may be used to design non-human adhesion protein, protease and protease inhibitor nucleic acids, fragments, variants and homologues. Mutagenesis may be carried out by means known in the art to produce further variety.

Sequences of homologues may be obtained by probing cDNA libraries made from or genomic DNA libraries from other animal species, and probing such libraries with probes comprising all or part of any of the adhesion protein, protease or protease inhibitor nucleic acids, fragments, variants and homologues, or other fragments under conditions of medium to high stringency.

Similar considerations apply to obtaining species homologues and allelic variants of the polypeptide or nucleotide sequences disclosed here.

Variants and strain/species homologues may also be obtained using degenerate PCR which will use primers designed to target sequences within the variants and homologues encoding conserved amino acid sequences within the sequences of the nucleic acids encoding adhesion proteins, proteases or protease inhibitors. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences. It will be appreciated by the skilled person that overall nucleotide homology between sequences from distantly related organisms is likely to be very low and thus in these situations degenerate PCR may be the method of choice rather than screening libraries with labelled fragments the sequences.

In addition, homologous sequences may be identified by searching nucleotide and/or protein databases using search algorithms such as the BLAST suite of programs.

Alternatively, such polynucleotides may be obtained by site directed mutagenesis of characterised sequences, for example, adhesion protein, protease and protease inhibitor nucleic acids, or variants, homologues, derivatives or fragments thereof. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The polynucleotides described here may be used to produce a primer, e.g. a PCR primer, a primer for an alternative amplification reaction, a probe e. g. labelled with a revealing label by conventional means using radioactive or non-radioactive labels, or the polynucleotides may be cloned into vectors. Such primers, probes and other fragments will be at least 8,9,10, or 15, preferably at least 20, for example at least 25, 30 or 40 nucleotides in length, and are also encompassed by the term "polynucleotides" as used herein.

Polynucleotides such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily-available in the art.

Longer polynucleotides will generally be produced using recombinant means, for example using a PCR (polymerase chain reaction) cloning techniques. This will involve making a pair of primers (e. g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e. g. by purifying the reaction mixture on an agarose gel) and recovering the amplified DNA. The primers may be designed to contain suitable restriction enzyme recognition sites so that the amplified DNA can be cloned into a suitable cloning vector.

Polynucleotides or primers may carry a revealing label. Suitable labels include radioisotopes such as ³²p or ³⁵S, enzyme labels, or other protein labels such as biotin. Such labels may be added to polynucleotides or primers and may be detected using by techniques knownper se. Polynucleotides or primers or fragments thereof labelled or unlabeled may be used by a person skilled in the art in nucleic acid-based tests for detecting or sequencing polynucleotides in the human or animal body.

Such tests for detecting generally comprise bringing a biological sample containing DNA or RNA into contact with a probe comprising a polynucleotide or primer under hybridising conditions and detecting any duplex formed between the probe and nucleic acid in the sample. Such detection may be achieved using techniques such as PCR or by immobilising the probe on a solid support, removing nucleic acid in the sample which is not hybridised to the probe, and then detecting nucleic acid which has hybridised to the probe. Alternatively, the sample nucleic acid may be immobilised on a solid support, and the amount of probe bound to such a support can be detected. Suitable assay methods of this and other formats can be found in for example W089/03891 and W090/13667.

Tests for sequencing nucleotides, involve bringing a biological sample containing target DNA or RNA into contact with a probe comprising a polynucleotide or primer under hybridising conditions and determining the sequence by, for example the Sanger dideoxy chain termination method (see Sambrook *et al.*)*.*

Such a method generally comprises elongating, in the presence of suitable reagents, the primer by synthesis of a strand complementary to the target DNA or RNA and selectively terminating the elongation reaction at one or more of an A, C, G or T/U residue; allowing strand elongation and termination reaction to occur; separating out according to size the elongated products to determine the sequence of the nucleotides at which selective termination has occurred. Suitable reagents include a DNA polymerase enzyme, the deoxynucleotides dATP, dCTP, dGTP and dTTP, a buffer and ATP. Dideoxynucleotides are used for selective termination.

### ANTIBODIES

We further provide an antibody, either polyclonal or monoclonal, which specifically binds to epitopes on the polypeptide/protein encoded by the corneodesmosin gene or mutant epitopes. In preparing the antibody, the protein (with and without mutations) encoded by the corneodesmosin gene and polymorphisms thereof is used as a source of the immunogen. Peptide amino acid sequences isolated from the amino acid sequence or mutant peptide sequences may also be used as an immunogen.

The antibodies may be either monoclonal or polyclonal. Conveniently, the antibodies may be prepared against a synthetic peptide based on the sequence, or prepared recombinantly by cloning techniques or the natural gene product and/or portions thereof may be isolated and used as the immunogen. Such proteins or peptides may be used to produce antibodies by standard antibody production technology well known to those skilled in the art as described generally in Harlow and Lane, Antibodies: A Laboratory Manual, Cold Spring Harbor Laboratory Press, Cold Spring Harbor, N. Y., 1988.

For producing polyclonal antibodies a host, such as a rabbit or goat, is immunized with the protein or peptide, generally with an adjuvant and, if necessary, coupled to a carrier; antibodies to the protein are collected from the sera.

For producing monoclonal antibodies, the technique involves hyperimmunization of an appropriate donor, generally a mouse, with the protein or peptide fragment and isolation of splenic antibody producing cells. These cells are fused to a cell having immortality, such as a myeloma cell, to provide a fused cell hybrid which has immortality and secretes the required antibody. The cells are then cultured, in bulk, and the monoclonal antibodies harvested from the culture media for use.

The antibody may be bound to a solid support substrate or conjugated with a detectable moiety or be both bound and conjugated as is well known in the art. (For a general discussion of conjugation of fluorescent or enzymatic moieties see Johnstone and Thorpe, Immunochemistry in Practice, Blackwell Scientific Publications, Oxford, 1982.) The binding of antibodies to a solid support substrate is also well known in the art. (see for a general discussion Harlow and Lane Antibodies: A Laboratory Manual, Cold Spring Harbor Laboratory Publications, New York, 1988) The detectable moieties may include, but are not limited to, fluorescent, metallic, enzymatic and radioactive markers such as biotin, gold, ferritin, alkaline phosphatase, β-galactosidase, peroxidase, urease, fluorescein, rhodamine, tritium, ¹⁴C and iodination.

### LINKAGE DISEQUILIBRIUM ANALYSIS

Linkage disequilibrium (LD) analysis is a powerful tool for mapping disease genes and may be particularly useful for investigating complex traits. LD mapping is based on the following expectations: for any two members of a population, it is expected that recombination events occurring over several generations will have shuffled their genomes, so that they share little in common with their ancestors. However, if these individuals are affected with a disease inherited from a common ancestor, the gene responsible for the disease and the markers that immediately surround it will likely be inherited without change, or IBD ("identical by descent"), from that ancestor. The size of the regions that remain shared (i. e. IBD) are inversely proportional to the number of generations separating the affected individuals and their common ancestor. Thus, "old" populations are suitable for fine scale mapping and recently founded ones are appropriate for using LD to roughly localize disease genes. (Houwen et al., 1994, in particular FIG. 3 and accompanying text). Because isolated populations have typically had a small number of founders, they are particularly suitable for LD approaches, as indicated by several successful LD studies conducted in Finland (de la Chapelle, 1993).

LD analysis has been used in several positional cloning efforts (Kerem et al., 1989; MacDonald et al., 1992; Petrukhin et al., 1993; Hastbacka et al., 1992 and 1994), but in each case the initial localization had been achieved using conventional linkage methods. Positional cloning is the isolation of a gene solely on the basis of its chromosomal location, without regard to its biochemical function. Lander and Botstein (1986) proposed that LD mapping could be used to screen the human genome for disease loci, without conventional linkage analyses. This approach was not practical until a set of mapped markers covering the genome became available (Weissenbach et al., 1992). The feasibility of genome screening using LD mapping is now demonstrated by the applicants.

Identification of the chromosomal location of a gene responsible for causing a disease associated with increased or reduced cell-cell adhesion in the epithelium can facilitate diagnosis, treatment and genetic counseling of individuals in affected families.

Due to the severity of the disorder and the limitations of a purely phenotypic diagnosis of such diseases, there is a tremendous need to genetically subtype individuals to confirm clinical diagnoses and to determine appropriate therapies based on their genotypic subtype.

### DIAGNOSIS OF DISEASES

We provide for methods of diagnosis of diseases associated with abnormal epithelial cell-cell adhesion. Mutations in genes encoding adhesion proteins (for example, corneodesmosin) are shown to result in reduced adhesion between epithelial cells such as corneocytes. Some mutations in these genes may result in altered (typically reduced) expression of adhesion proteins, or in expression of adhesion proteins which have reduced adhesion activity or higher protease sensitivity. Other mutations in protease and protease inhibitor genes are also associated with disease, as shown in the Examples. Such mutations are typically associated with and may lead to Group I diseases, as the reduced cell-cell adhesion results in impaired barrier function of the epidermis.

Accordingly, a Group 1 disease may be diagnosed in a patient suffering or likely to suffer from the disease by determining the level of expression of an adhesion protein, in which a reduced level of expression of an adhesion protein is diagnostic of a Group 1 disease. Furthermore, we provide a method of diagnosis of a Group 1 disease by determining the adhesion activity of an adhesion protein involved in epithelial cell- cell adhesion, in which a reduced adhesion activity is diagnostic of a Group 1 disease. We further provide a method of diagnosis of a Group 1 disease by determining the protease sensitivity of an adhesion protein involved in epithelial cell-cell adhesion, in which an increased protease sensitivity is diagnostic of a Group 1 disease. We also disclose a method of diagnosis of a Group 1 disease, by determining the presence of a mutation which is associated with a reduced expression of an adhesion protein, or with expression of an adhesion protein with reduced adhesion activity and/or increased protease sensitivity.

According to a preferred embodiment, diagnosis of a Group 1 disease is carried out by detection of the absence of a *Hph*l restriction enzyme site in the corneodesmosin gene.

Preferably, the Group 1 disease diagnosed by the absence of an Hphl site is eczema or Crohn's disease.

Alternatively or in conjunction, we provide for the diagnosis of a Group 1 disease by detection of the presence of a T at position at +1243 in the corneodesmosin gene. Preferably, the Group 1 disease diagnosed by the presence of a T at position +1243 is eczema or Crohn's disease.

Preferably, the presence of T at position +1243 is associated with a C to T transition.

Any method of determining protease sensitivity of a protein as known in the art (Egelrud, 1993) may be used in the diagnostic methods presented above. Similarly, detection of mutations by means of, for example, SSCP, RFLP, SNP, etc analysis are known in the art and are described in further detail below.

Detection of abnormal (i. e., increased) proteolytic breakdown of an adhesion protein may also be used to diagnose Group 1 diseases. Thus, an increased level of expression and/or activity of a protease involved in proteolysis of an adhesion protein is characteristic (and hence diagnostic) of a Group 1 disease. Accordingly, we provide a method of diagnosis of a Group 1 disease, by detecting the level of expression of a protease involved in proteolysis of an adhesion protein. We further provide a method of diagnosis of a Group 1 disease, by detecting the level of activity of a protease involved in proteolysis of an adhesion protein, in which an increased level of activity of the protease is diagnostic of a Group 1 disease.

The level of expression and/or activity of a protease inhibitor which is capable of inhibiting the proteolytic activity of a protease involved in breakdown of an adhesion protein may also be detected as a means of diagnosis of a Group 1 or Group 2 disease. Accordingly, an decreased level of expression and/or activity of a protease inhibitor is characteristic of a Group 1 disease. Therefore, a method of diagnosis of a Group 1 disease comprises detecting a decreased level of expression of a protease inhibitor in a patient. Similarly, a method of diagnosis of a Group 1 disease may also comprise detecting the level of activity of a protease inhibitor, in which an decreased level of activity of a protease inhibitor is diagnostic of a Group 1 disease.

It is clear that detection of expression and/or activity of proteases and/or protease inhibitors may also be undertaken at a genetic level, i. e., detecting mutations associated with increased or decreased protease/protease inhibitor activity.

Preferably, the adhesion protein which is detected or whose properties are determined in the above methods is corneodesmosin, and preferably, the gene is a gene encoding corneodesmosin. Preferably, the protease that is detected, etc is stratum corneum chymotryptic enzyme (SCCE) or stratum corneum tryptic enzyme (SCTE).

Preferably, the protease inhibitor that is detected, etc, is anti-leukoprotease (SLPI) or elafin protease inhibitor 3 (PI3 or SKALP).

Preferably, the protease inhibitor that is detected, etc, is or elafin. Where the terms "higher", "increased", and "enhanced" are used with reference to activity, protease sensitivity, expression, etc, these are understood to be relative to the corresponding properties of a normal, un-diseased epithelium from the same patient or another individual. Similarly, the terms "lower", "decreased" and "reduced" are to refer to a level relative to a normal, un-diseased epithelium.

Specific non-limiting examples of diagnostic methods suitable for the methods and compositions described here are set out below:

Genetic diagnostic tests may be performed by genotyping genes encoding corneodesmosomal proteins (e. g. comeodesmosome), proteases (SCCE, SCTE) and protease inhibitors (i. e. SKALP, SLPI) for SNPs associated with disease of interest. Primers are designed correspond to sequences which flank mutations and/or polymorphisms. The sequences of proteases (SCCE, SCTE) and protease inhibitors (i. e. SKALP, SLPI) genes may, for example, be used to design primers in the extremities of each exon using Gene Jockey II software (Biosoft, 49 Bateman Street, Cambridge CB2 1LR). After PCR amplification, the mutation can may be detected by allelic discrimination using restriction enzymes, TaqMan analysis (as described below) or simply by sequencing.

Briefly, 25µl PCR reactions comprised 8% glycerol, 200 µM each dATP, dGTP and dCTP, 400 µM dUTP, 1.25 U AmplitaqGold (Perkin-Elmer, U. S. A), 1.25 U Uracil-N-Glycosylase (Perkin-Elmer, USA), 5mM MgCl₂, 500-900 nM each primer. Allelic discrimination at these loci is performed using a 5' nuclease assay (TaqMan^{™} allelic discrimination test), a method extensively validated. This test is based on 5' nuclease activity of Taq polymerase and the detection by fluorescence-resonance energy transfer (FRET) of the cleavage of two probes designed to hybridise to either allele during PCR. Double fluorescent probes are provided by ABI-PE (Forster City, CA; Warrington, UK). Probe and primer sequences are designed and probes are labelled with carboxyfluorescein (FAM) and carboxy-4,7,2' 7'-tetrachlorofluorescein (TET) fluorescent dyes at the 5'end, and with the quencher carboxytetramethylrhodamine (TAMRA) at the 3'terminus. Concentrations of FAM and TET probes ranged between 20-50 nM and 50-350 nM respectively depending on the probes used. Plates are scanned in an LS50-B or a PE7200 fluorimeter (ABI/Perkin-Elmer).

As used herein the term "polymerase chain reaction" or "PCR" refers to the PCR procedure described in the patents to Mullis, et al., U. S. Pat. Nos. 4, 683, 195 and 4,683,202. The procedure basically involves: (1) treating extracted DNA to form single-stranded complementary strands; (2) adding a pair of oligonucleotide primers, wherein one primer of the pair is substantially complementary to part of the sequence in the sense strand and the other primer of each pair is substantially complementary to a different part of the same sequence in the complementary antisense strand; (3) annealing the paired primers to the complementary sequence; (4) simultaneously extending the annealed primers from a 3' terminus of each primer to synthesize an extension product complementary to the strands annealed to each primer wherein said extension products after separation from the complement serve as templates for the synthesis of an extension product for the other primer of each pair; (5) separating said extension products from said templates to produce single-stranded molecules; and (6) amplifying said single-stranded molecules by repeating at least once said annealing, extending and separating steps.

Biochemical diagnostic tests include proteolysis profiles, screening for N/C terminals, screening for enzyme function and/or activity.

Proteolysis profiles may be conducted as follows. Polyclonal antibody capable of recognising the full and the mature forms of corneodesmosin are generated. Stratum corneum extract may be extracted from strips obtained from patients. Protein extracts are run on SDS-PAGE gel, transferred onto a membrane and hybridised with primary anti-corneodesmosin antibody and labelled secondary antibody. Molecular weight of processed corneodesmosin of patients are compared to the ones from controls.

Screening for N-/C-terminals may be conducted as follows. In the case of the presence of particular forms of desmosomal/corncodesmosomal proteins (e. g. corneodesmosin) in a patient group but not in controls or vice versa, N-/C-terminal specific antibodies antibody may be produced to detect the pathogenic forms of desmosomal/corneodesmosomal proteins (e. g. corneodesmosin). The identification of the pathogenic forms may be performed as follow. Different forms of corneodesmosin are purified from non denaturing hypotonic buffer extract (TEA buffer extract) of human epidermis by anion exchange and affinity chromatography (Simon et al, 1997). The eluted fractions of the affinity column are pooled, lyophilized, separated by SDS- PAGE. The bands corresponding to the pathogenisis forms of corneodesmosin are excised and characterized by internal and NH2-terminal amino acid sequence analysis.

Screening for enzyme function/activity may be conducted as follows.

Desmosomal/corneodesmosomal proteins (e.g. corneodesmosin) are radiolabelled during *in vitro* translation from cDNA and incubated with protease enzymes (SCCE, SCTE) at 37°C for 1.5-3 h. SCCE and SCTE may be prepared using KC 1 extract of dissociated plantar corneocytes as described by Egelrud, 1993, and Brattsand and Egelrud, 1999 (Egelrud, 1993; Brattsand and Egelrud, 1999). SCCE may be purified by affinity chromatography on SBTI Affigel 15: An alternative method is to produce a fusion protein *in vitro* and purify using immunoaffinity columns (Ekholm et al, 2000). SDS-PAGE and autoradiography are used to analyze the pattern of- hydrolytic peptides. The intensity of autoradiography bands is quantified by a densitometry and the fraction of peptides less that 56 kDa (full length) is calculated. Activity is expressed as mol substrate processed by g enzyme per second.

### DESMOSOMES AND CORNEODESMOSOMES

Desmosomes are symmetrical structures that form disc-shaped intercellular junctions between epithelial cells. In the epidermis the desmosomes mediate the adhesion between keratinocytes. In psoriasis, various ichtyoses and skin xerosis, the number of corneodesmosomes (desmosomes in upper layers of the epidermis) is increased in the stratum corneum. Immunoelectron microscopy has been used to show the define the interactions within corneodesmosomes between proteins of the extracellular core domain such as desmoglein and desmocollins and intracellular corneodesmosomal proteins including desmoplakins I and II, plakoglobin (PG) and plakophilins (PP) (Cowin and Burke, 1996). The importance of corneodesmosomal proteins in epidermal integrity is demonstrated by inherited disorders such as striate subtype of palmoplantar keratoderma caused by desmoplakin haploinsufficiency (Armstrong et al, 1999). Mutations in loricrin gene lead to keratoderma of Camisa. Corneodesmosin is a glycoprotein of corneodesmosomes. Three forms of the corneodesmosin with different weights 33-36 to 40-46 and 52-56 kDa have been isolated from the epidermis (Simon et al, 1997).

Mutations within the corneodesmosin/S gene and related genes within the MHC epidermal gene cluster (chromosome 6p21) result in a reduced cohesion between corneocytes.

### GENETIC DIAGNOSIS

We disclose a method for diagnosing and detecting carriers of a defective an adhesion protein, protease or protease inhibitor gene responsible for causing Group 1 or Group 2 diseases, or associated with abnormal regulation of proteolysis of an adhesion protein such as corneodesmosin.

We further provide methods for detecting normal copies of the gene and its gene product. Identifying carriers either by their defective gene or by their missing or defective protein (s) encoded thereby, leads to earlier and more consistent diagnosis of gene carriers. Thus, since carriers of the disease are more likely to be prone to Group 1 or Group 2 diseases, better surveillance and treatment protocols may be initiated for them.

Briefly, the methods comprise the steps of obtaining a sample from a test subject, isolating the appropriate test material from the sample and assaying for the target nucleic acid sequence or gene product. The sample may be tissue or bodily fluids from which genetic material and/or proteins are isolated using methods standard in the art. For example, DNA may be isolated from blood.

More specifically, the method of carrier detection is carried out by first obtaining a sample of either cells or bodily fluid from a subject. Convenient methods for obtaining a cellular sample may include collection of either mouth wash fluids or hair roots. A cell sample could be amniotic or placental cells or tissue in the case of a prenatal diagnosis. A crude DNA could be made from the cells (or alternatively proteins isolated) by techniques well known in the art. This isolated target DNA is then used for PCR analysis (or alternatively, Western blot analysis for proteins) with appropriate primers derived from the gene sequence by techniques well known in the art. The PCR product would then be tested for the presence of appropriate sequence variations in order to assess genotypic disease status of the subject.

The specimen may be assayed for polypeptides/proteins by immunohistochemical and immunocytochemical staining (see generally Stites and Terr, Basic and Clinical Immunology, Appleton and Lange, 1994), ELISA, RIA, immunoblots, Western blotting, immunoprecipitation, functional assays and protein truncation test. In preferred embodiments, Western blotting, functional assays and protein truncation test (Hogervorst et al., 1995) are used. mRNA complementary to the target nucleic acid sequence may be assayed by in situ hybridization, Northern blotting and reverse transcriptase-polymerase chain reaction. Nucleic acid sequences may be identified by in situ hybridization, Southern blotting, single strand conformational polymorphism, PCR amplification and DNA-chip analysis using specific primers. (Kawasaki, 1990; Sambrook, 1992; Lichter *et al*, 1990; Orita *et al*, 1989; Fodor *et al.*, 1993; Pease *et al.*, 1994)

ELISA assays are well known to those skilled in the art. Both polyclonal and monoclonal antibodies may be used in the assays. Where appropriate other immunoassays, such as radioimmunoassays (RIA) may be used as are known to those in the art. Available immunoassays are extensively described in the patent and scientific literature. See, for example, U. S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521 as well as Sambrook et al, 1992.

Current mutation data as described here indicate that Group 1 diseases are characterized by allelic heterogenicity. Thus, it is important for a successful mutation screen to be able to detect all possible nucleotide alterations in the corneodesmosin or other adhesion protein, protease or protease inhibitor gene, rather than being focused on a limited subset. Methods including direct sequencing of PCR amplified DNA or RNA or DNA chip hybridization (Fodor *et al.*, 1993; Pease *et al.*, 1994) may be applied along with other suitable methods known to those skilled in the art.

In order to use the methods for diagnostic applications, it is advantageous to include a mechanism for identifying the presence or absence of target polynucleotide sequence (or alternatively proteins). In many hybridization based diagnostic or experimental procedures, a label or tag is used to detect or visualize for the presence or absence of a particular polynucleotide sequence. Typically, oligomer probes are labelled with radioisotopes such as 32 P or 35 S (Sambrook, 1992) which may be detected by methods well known in the art such as autoradiography. Oligomer probes may also be labelled by non-radioactive methods such as chemiluminescent materials which may be detected by autoradiography (Sambrook, 1992). Also, enzyme-substrate based labelling and detection methods may be used. Labelling may be accomplished by mechanisms well known in the art such as end labelling (Sambrook, 1992), chemical labelling, or by hybridization with another labelled oligonucleotide. These methods of labelling and detection are provided merely as examples and are not meant to provide a complete and exhaustive list of all the methods known in the art.

The introduction of a label for detection purposes may be accomplished by attaching the label to the probe prior to hybridization.

An alternative method includes the step of binding the target DNA to a solid support prior to the application of the probe. The solid support may be any material capable of binding the target DNA, such as beads or a membranous material such as nitrocellulose or nylon. After the target DNA is bound to the solid support, the probe oligomers is applied.

Functional assays may be used for detection of Group 1 carriers or affected individuals.

We also provide a kit for diagnosis and detection of a defective corneodesmosin gene. The kit includes a molecular probe complementary to genetic sequences of a defective corneodesmosin gene which causes a Group 1 disease and suitable labels for detecting hybridization of the molecular probe and the defective gene thereby indicating the presence of the defective gene. The molecular probe has a DNA sequence complementary to mutant sequences. Alternatively, the kit may contain reagents and antibodies for detection of mutant proteins.

A kit for detection and diagnosis of a defective adhesion protein, protease or protease inhibitor gene is also provided, comprising a molecular probe complementary to genetic sequences of a defective adhesion protein, protease or protease inhibitor gene which causes a Group 1 disease and suitable labels for detecting hybridization of the molecular probe and the defective gene thereby indicating the presence of the detective gene. The molecular probe has a DNA sequence complementary to mutant sequences. Alternatively, the kit may contain reagents and antibodies for detection of mutant proteins.

A few different methods are commonly used to analyze DNA for polymorphisms or mutations. The most definitive method is to sequence the DNA to determine the actual base sequence (Maxam and Gilbert, 1977; Sanger et al., 1977). Although such a method is the most definitive it is also the most expensive and time consuming method.

Restriction mapping analysis may also be used in analyzing DNA for polymorphisms. If one is looking for a known polymorphism at a site which will change the recognition site for a restriction enzyme it is possible simply to digest DNA with this restriction enzyme and analyze the fragments on a gel or with a Southern blot to determine the presence or absence of the polymorphism. This type of analysis is also useful for determining the presence or absence of gross insertions or deletions. Hybridization with allele specific oligonucleotides is yet another method for determining the presence of known polymorphisms.

Thus, specific DNA sequences in an individual, for example, a gene encoding corneodesmosin, may undergo many different changes, such as deletion of a sequence of DNA, insertion of a sequence that was duplicated, inversion of a sequence, or conversion of a single nucleotide to another. Changes in a specific DNA sequence may be traced by using restriction enzymes that recognize specific DNA sequences of 4-6 nucleotides. Restriction enzymes cut (digest) the DNA at their specific recognized sequence, resulting in one million or so pieces. When a difference exists that changes a sequence recognized by a restriction enzyme to one not recognized, the piece of DNA produced by cutting the region are of a different size. The various possible fragment sizes from a given region therefore depend on the precise sequence of DNA in the region. Variation in the fragments produced is termed"restriction fragment length polymorphism" (RFLP). The different sized-fragments reflecting different variant DNA sequences may be visualized by separating the digested DNA according to its size on an agarose gel and visualizing the individual fragments by annealing to a radioactively labeled DNA "probe". Each individual may carry two different forms of the specific sequence. When the two homologues carry the same form of the polymorphism, one band is seen. More than two forms of a polymorphism may exist for a specific DNA marker in the population, but in one family just four forms are possible; two from each parent. Each child inherits one form of the polymorphism from each parent. Thus, the origin of each chromosome region may be traced (maternal or paternal origin).

Furthermore, RT-PCR may be carried out, followed by restriction endonuclease fingerprinting (REF). REF is a modification of the single-strand conformation polymorphism (SSCP) method, and enables efficient detection of sequence alterations in DNA fragments up to 2 kb in length (Liu and Sommer, 1995).

The use of mass spectrometry to determine the presence of polymorphisms within known genes is disclosed in United States Patent No. 5, 869, 242.

Single strand conformational polymorphism (SSCP) analysis is a rapid and efficacious method for detecting polymorphisms (Dean et al., 1990; Glavac and Dean,1993; Poduslo *et al.*, 1991). In SSCP, abnormal strand motility on a gel is associated with mutational events in the gene.

Genetic analysis of polymorphisms is disclosed in detail in United States Patent Nos. 5,552,28, 5,654,13, 5,670,33, 5,807,67, 5,858,66, 5,691,15, 5,922,57, 5,972,60, 6,36,53 and 5,955,26, among others.

Any of these methods described in the above references may be used in the diagnostic methods described here.

### EXAMPLES

### EXAMPLES A: ADHESION PROTEIN POLYMORPHISMS

### Example Al. Identification of Corneodesmosin (S) Gene Polymorphisms

Genomic DNA is extracted from whole blood according to standard protocols and stored at 100 ng/µl. One reported exonic polymorphism giving a T to C transition at position +1243 is analysed (Ishihara *et al*, 1996, Tazi-Ahnini et al, 1999a, 1999b).

Primers S 15 (5'CATTGCATTCCAGCCAGTGG3') and S 16
(5'AACTGGAGCTGCTGCTGAAGGA3') are used to amplify the polymorphism locus (+1243). PCRs are prepared in bulk and aliquoted to 25 µl volumes comprising 50 mM KCL, 20 mM Tris-HCL, 1.5 mM MgCl₂, 200 µM each dNTP, 1.2 M each primer, 1 U of *Taq* polymerase (Gibco BRL, Paisley, UK) and 200 ng of genomic DNA. Thermocycling conditions arc 2 min at 95°C, 28 cycles of 1 min at 95°C, 1 min at 58°C and 15 s at 72°C. The amplifications are ended by 15 min at 72°C.

Restriction digests are performed in 20 µl reactions containing 10 µl of PCR products and 2.5 U of *Hph*I and appropriate manufacturer's buffer (New England Biolabs, Hitchin, UK) at 37°C overnight. Allelic discrimination is performed by electrophoresis using 2% agarose. *Hph*I digestion produces 123 + 89 bp for allele 1, while it does not cut allele 2 (212 bp).

### Alleles

In the following Examples, "allele 1" refers to an allele in which the nucleotide residue at position + 1243 is a C. The protein encoded by such an allele has a serine (S) residue at position 394 of the amino acid sequence employing the numbering of the polypeptide sequence having accession number L20815.

Similarly, "allele 2" refers to an allele in which the nucleotide residue at position +1243 is a T. The protein encoded by such an allele has a leucine residue at position 394 of a amino acid sequence, employing the numbering of the polypeptide sequence having accession number L20815.

Amino acid position numbering refers to the translation initiation codon (+1, ATG) of corneodesmosin sequence with GenBank accession numbers GB: L20815 or AF030130, as indicated. Thus, for example, a +1243 substitution C to T gives amino acid change from S to F at position 394 according to GB sequence L20815, while the same nucleotide substitution gives amino acid change from S to F at position 410 according to the GB sequence AF030130.

### Statistical Analysis

Disease and control populations are compared using 2x3 tables, and the odds ratio is calculated by comparing individuals homozygotic for allele 1 with that for carriage of the alternative allele (allele 2) in control and patients population. A_{χ}² test for allele 2 is also carried out, weighted by number of putative disease susceptibility alleles in each genotype group.

### Example A2. Association of Corneodesmosin (S) Gene +1243 Polymorphisms with Atopic Eczema

In this Example, unless otherwise indicated, amino acid positions in the corneodesmosin sequence are provided with reference to the numbering in GenBank sequence L20815.

This Example demonstrates that corneodesmosin allele 2, in which the nucleotide residue of corneodesmosin at position +1243 is a T, leading to the presence of a leucine (T) residue at position 394 of the corneodesmosin polypeptide (L20815), is associated with atopic eczema.

The allelic distribution of + 1243 polymorphism is assessed in both the atopic eczema and controls groups (n=154 and 550, respectively), as described above. Both controls and patients are in Hardy Weinberg equilibrium. Table A2.1 shows the observed (A) and the expected (B) values of each genotype in controls and atopic eczema patients groups.

**Table A2.1: Allelic distribution of allele 11,12 and 22 in control and atopic eczema patient groups. A: Observed values; B: Expected Values**

| (A) *Observed values* | | | |
|---|---|---|---|
| | 22 (T/T) | 12 (T/C | 11 (C/C) |
| Controls | 150 | 284 | 116 |
| Patients | 52 | 74 | 28 |

| (B) *Expected values* | | | |
|---|---|---|---|
| | 22 (T/T) | 12 (T/C) | 11 (C/C) |
| Controls | 157.8 | 279.7 | 112.5 |
| Patients | 44.2 | 78.3 | 31.5 |

The overall difference between expected and observed values in the controls and patients is not statistically significant. However, there is an increase of the rare allele (allele 2, 52/44.2=1.2). Allele 1 (common allele) appears to be protective from eczema, and for this reason we group individuals with alleles 12 and 11 in one subgroup and 22 in the other (see Table A2.2 below).

**Table A2.2: Allelic distribution of allele 11 and 12/22 in control and atopic eczema patient groups**

| | 22 (TT) | 11/12 (CC/CT) |
|---|---|---|
| Patients | 52 | 102 |

A χ² test for allele 2 against carriage of allele 1 is carried out. A significant association is found between allele 2 of the corneodesmosin gene (+1243) polymorphism and atopic eczema [OR = 1.36 (0.93,1.99)].

We therefore disclose the diagnosis of a Group I disease or susceptibility to a Group I disease (preferably eczema or susceptibility to eczema, preferably atopic eczema) in an individual, by detecting the presence of a T at position + 1243 of a corneodesmosin nucleic acid, or the presence of a leucine (L) residue at position 394 of a corneodesmosin polypeptide (L20815), or both, of an individual.

### Example A3. Association of Corneodesmosin (S) Gene +1243 Polymorphisms with Dermatitis

In this Example, unless otherwise indicated, amino acid positions in the corneodesmosin sequence are provided with reference to the numbering in GenBank sequence L20815.

This Example demonstrates that corneodesmosin allele 2, in which the nucleotide residue of corneodesmosin at position +1243 is a T, leading to the presence of a leucine (L) residue at position 394 of the corneodesmosin polypeptide (L20815), is associated with dermatitis and might be the cause of the pathogenesis of dermatitis herpetiformis.

The allelic distribution of the +1243 polymorphism is assessed in both the dermatitis herpetiformis and controls groups (n=50 and 550, respectively), as described above. Both controls and patients are in Hardy Weinberg equilibrium.

**Table A3.1: Allelic distribution of allele 11,12 and 22 in control and dermatitis herpetiformis groups**

| A) Observed values | | | |
|---|---|---|---|
| | 22 (T/T) | 12 (T/C) | 11 (C/C) |
| Controls | 150 | 284 | 116 |
| Patients | 26 | 23 | 1 |

| B) Expected values | | | |
|---|---|---|---|
| | 22 (T/T) | 12 (T/C) | 11 (C/C) |
| Controls | 161.3 | 281. 4 | 107.2 |
| Patients | 14. 6 | 25.5 | 9. 7 |

There is an increase of the rare allele (allele 2,26/14.6=1.78). Only one patient is homozygous for allele 1 (a common allele). This allele seems to be a protective since it is very frequent in control population.

**Table A3.2: Allelic distribution of allele 11 and 12/22 in control and dermatitis herpetiformis groups**

| | TT/CT (22/12) | CC (12/11) |
|---|---|---|
| Controls | 434 | 116 |
| Patients | 49 | 1 |

A χ² test for allele 1 against carriage of allele 2 is carried out. A significant association is found between allele 2 of S gene (+1243) polymorphism and dermatitis herpetiformis [OR= 13.10 (1.79,95.85); p<0.0001].

We therefore disclose the diagnosis of a Group I disease or susceptibility to a Group I disease (preferably dermatitis or susceptibility to dermatitis, preferably dermatitis herpetiformis) in an individual, by detecting the presence of a T at position +1243 of a corneodesmosin nucleic acid, or the presence of a leucine (L) residue at position 394 of a corneodesmosin polypeptide (L20815), or both, of an individual. This amino acid is at position 410 according to the GB sequence AF030130.

Allele 2 or T at position +1243 confers higher risk for dermatitis herpetiformis compared to atopic eczema because individual heterozygous at +1243 have also high risk of developing dermatitis.

### Example A4. Linkage Disequilibrium Analysis

In this Example, unless otherwise indicated, amino acid positions in the corneodesmosin sequence are provided with reference to the numbering in GenBank sequence L20815.

It has previously been shown that C at position +1243 is in linkage disequilibrium with T and G at position +619 and + 1240 respectively (Tazi-Ahnini et al, 1999a ; Allen et al, 1999). The linkage disequilibrium is extended to other loci within the coding sequence of the corneodesmosin. Furthermore, using pedigree analysis with the transmission disequilibrium test (TDT), Jenisch and his colleagues identify 6 different alleles encoding 6 different amino acid sequences of the corneodesmosin gene (Jenisch et al, 1999).

### Group I Diseases

We show that within allele 5 (CD5) and allele 6 (CD6), T (leu) at position +1243 is in strong linkage disequilibrium with A (asp), AGT (ser), T (phe), A (ser), T (ser), T (leu), G (asp) and T (asn) of CD5 and A (asp), deletion (-), T (phe), A (ser), T (ser), T (leu), G (asp) and C (asp) of CD6. These variants are at position +442, + 468, +619, +1215, +1236, +1243, +1515 and +1593 respectively.

We find a strong association of these variants with atopic eczema in our collection. Therefore, detection of any of the changes listed above, and shown to be in linkage disequilibrium with T at position +1243 (i.e., allele 2), may be used in place of, or in addition to, detection of nucleotide T (+1243) or amino acid L (394 of L20815).

We therefore provide the diagnosis of a Group I disease or susceptibility to a Group I disease in an individual, preferably eczema or dermatitis, more preferably atopic eczema or dermatitis herpetiformis, or susceptibility to any of these diseases, by detecting any one or more of these changes.

Accordingly, we provide the diagnosis of a Group I disease, or susceptibility to a Group I disease, by detecting any one or more of the following residues at the relevant positions of a corneodesmosin nucleic acid, as shown in the Table A4.1 below.

| | | | | | | | | |
|---|---|---|---|---|---|---|---|---|
| Nucleotide Position | 442 | 468 | 619 | 1215 | 1236 | 1243 | 1515 | 1593 |
| Nucleic acid (s) | A | AGT | T | A | T | T | G | T |
| Residue Position (1) | 127 | 137 | 186 | 385 | 392 | 394 | 485 | 511 |
| Residue Position (2) | 143 | 153 | 202 | 401 | 408 | 410 | 501 | 527 |
| Residue | D | S/- | F | S | S | L | D | D/N |

### Table A4.1. Corneodesmosin Polymorphisms and Group I Diseases. (1) : amino acid position numbering according to GenBank sequence L20815; (2): amino acid position numbering according to GenBank sequence AF030130

The above nucleic acid changes lead to changes in the amino acid sequence of corneodesmosin. Accordingly, the detection of the nucleotide residues at the relevant nucleic acid positions may also be achieved by detecting their effects, i.e., detecting a corresponding change in the encoded polypeptide sequence. Thus, for example, instead of or in addition to detecting a polymorphism at position +1243 of the nucleic acid sequence using specific nucleic probe or digestive enzyme (e. g. HphI), monoclonal antibodies may be used which are able to discriminate between a L residue at position 394 and an S residue at position 394.

We also provide the diagnosis of a Group I disease, or susceptibility to a Group I disease, by detecting any one or more of the following residues at the relevant positions of a corneodesmosin polypeptide, as shown in the Table A4.1.

### Summary

We have shown an association of a marker within the corneodesmosin gene giving an increased susceptibility to atopic eczema and dermatitis herpetiformis. It should be mentioned here that the corneodesmosin allele having C at +1243 (allele 1) is associated with psoriasis and acne vulgaris, suggesting that the two alleles give rise to different susceptibilities. Allele 2 (L394) is associated to barrier dysfunction (e. g. dermatitis, Crohn's disease) and allele 1 (S394) to impaired desquamation (e. g. psoriasis, acne).

We show association of polymorphisms at +1243 of the corneodesmosin gene with atopic eczema and dermatitis. The substitution at position +1243 gives amino acid change L394S. Without wanting to be bound by theory, we believe that this substitution interferes with the processing of corneodesmosin, thus contributing to enhanced desquamation.

We find that the corneodesmosin polymorphisms giving amino acid changes (Ser143/Asp, Ser153/-, Ser202/Phe, Ser401/Gly, Ser408/Ala, S410/L, Asp527/Asn ; position according to GB sequence AF030130) or (Ser127/Asp, Ser137/-, Ser186/Phe, Ser385/Gly, Ser392/Ala, S394/L, Asp511/Asn ; position according to GB sequence L20815) have an important function in the keratinocyte maturation and desquamation process. The screening method for these polymorphisms is described by Jenisch et al, 1999 and Guerrin et al, 2001. We therefore demonstrate that there is a strong relationship between the proteolytic processing of the corneodesmosin and the sensitivity of normal skin, and the integrity of diseased skin where there is a disruption in the barrier function including psoriasis, acne and dermatitis.

### Example A5. Corneodesmosin (S) Gene Polymorphism at Position 180

In this Example, unless otherwise indicated, amino acid positions in the corneodesmosin sequence are provided with reference to the numbering in GenBank sequence L20815.

### Identification of + 180 Corneodesmosin Gene Polymorphism

The S gene variant at position 180 is identified by automatic sequencing of a cloned S allele (Guerrin et al, 2001). Nucleic acid change from C to T at position +180 gives an amino acid change from L at position 40 (L20815) to F. This amino acid is very conserved in mammalian species. Sequence alignments of human, mouse and pig S sequences show that L40 is conserved between these species as detailed below in Table A5.1.

**Table A5. 1. Alignment of Corneodesmosin Sequences. Amino acid number is provided with reference to the numbering of the sequence in GenBank accession number L20815. Nucleic acid substitution C to T at position 180 gives amino acid change L to F at position 56 of the corneodesmosin protein according to GB sequence AF030130.**

| | |
|---|---|
| Mouse | ITSPNDPCLI |
| Pig1 | IASPNDPCLL |
| Pig2 | IASPSDPCLL |
| Human | ITSPNDPCL₄₀T |

### Chymotrypsin Proteolysis

Peptides corresponding to fragments of corneodesmosin, and comprising amino acids at and around position 40 (L20815; corresponding to position 180 on the nucleotide sequence) are synthesised. The peptides are exposed to chymotrypsin in appropriate buffer, and the digestion products identified. Chymotrypsin is known to cleave the peptide bond C-terminal to a F, Y or W residue, but not C-terminal to an L residue.

Peptides P1 and P2 (size 38 amino acids, comprising L and F at position 40 of L20815 respectively) are exposed to chymotrypsin. Peptide PI has the sequence PTRITSPNDPCL₄₀TGKGDSSGFSGSSSSGSSISSAR, while peptide P2 has the sequence PTRITSPNDPCF₄₀TGKGDSSGFSGSSSSGSSISSAR.

It is found that peptide P1 with L at position 40 of L20815 gives two products of proteolysis: PTRITSPNDPCL₄₀TGKGDSSGF and SGSSSSGSSISSAR. However, peptide P2 with F at position 40 of L20815 gives three small peptides PFRITSPNDPCF, TGKGDSSGF and SGSSSSGSSISSAR. The above results are confirmed by using the ExPASy Molecular Biology Server at http ://www.expasy. ch/. Peptides having the P 1 and P2 sequences are predicted to be cleaved differently by chymotrypsin.

This demonstrates that the L to F amino acid change creates a new chymotrypsin site within corneodesmosin. This has an important effect on the corneodesmosin maturation during keratinocyte differentiation and desquamation. We therefore disclose an association of a L to F amino acid polymorphism in corneodesmosin and a disease phenotype.

We therefore disclose a method for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of a protease cleavage site in a corneodesmosin polypeptide of an individual. Detection of the protease site may be done on the polypeptide sequence, or detecting a nucleic acid change which encodes a protease site.

Preferably, the protease cleavage site is a cleavage site of SCCE or SCTE.

### Group I Diseases

We find in particular that the amino acid substitution from L to F at position 40 is associated with Group I disease (e.g., eczema and/or dermatitis). This substitution is caused by a change from C to T at position 180 of the corneodesmosin nucleic acid.

We therefore provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of an F at position 40 of a corneodesmosin polypeptide of an individual.

We further provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of a T at position 180 of a corneodesmosin nucleic acid of an individual.

### Example A6. Identification of +619 Corneodesmosin Gene Polymorphism

We demonstrate that the nucleic acid substitution C to T at position +619 gives an amino acid change from S to F at position 186 according to the numbering in L20815 and position 202 according to the numbering in AF030130. This creates a new chymotryptic site within the corneodesmosin protein. We find that the serine at position 186 is very conserved in mammalian species.

We further demonstrate that F at position 186 is associated with detective skin barrier such as in eczema and dermatitis. F at position 186 is found in CD5 and CD6 of the corneodesmosin polypeptide.

### Group I Diseases

We therefore provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of an F at position 186 of a corneodesmosin polypeptide of an individual.

We further provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of a T at position 619 of a corneodesmosin nucleic acid of an individual.

### EXAMPLES C: PROTEOLYSIS PROFILES/PROTEASE MEDIATED MATURATION OF DESMOSOMAL AND CORNEODESMOSOMAL PROTEINS

### Example Cl. Production of Recombinant SCTE

Stratum Corneum Tryptic Enzyme (SCTE) is cloned and expressed in an active form such that it has full enzymatic activity.

### Expression Vectors Used

Expression is achieved using different vector systems in order to achieve protein expression at a high level, and enable purification. Three vector systems are used
*Insect Cell System*: No His-Tag at terminus; Invitrogen vector-pMT/V5-HisA, B or C are used, depending on the reading frame. Expression in S2 insect (Drosophila) cells; purification as described in Hansson et al., 1994.
*Retroviral System*: No His-Tag; Clontech retroviral vectors (pLNCX2) Protein is expressed in murine mammary cells, and purified as described in Hansson *et al.*, 1994.
*pcDNA3.1 System.* His-Tag at the C-terminal ; Invitrogen vectors (pcDNA3. 1, ABC with three ORFs) used. Expression of protein is achieved in COS-7 cells (Monkey African Green Kidney Cells). Reverse Transcription-polymerase Chain Reaction (RT-PCR)
PCR Primers, covering the open reading frame, are designed manually i. e. by sight using SCTE sequence (GB: AF168768). All primers are from Invitrogen
***Forward*** (5'TO 3'): GGA AAT CAG GTG CAG CG
***Reverse*** (5'TO 3'): GAT GAC TCA GGA GTT GGC
Human epidermis total RNA ISreverse transcribed to cDNA with the Applied Biosystems Gold RNA PCR Kit. The RT Cycle comprises hybridisation for 10 mins at 25°C, followed by Reverse Transcription for 12mins at 42 °C. PCR is performed using Applied Biosystems Gold Taq Polymerase with 2.0mM Mg²⁺, 40 cycles. Each PCR Cycle consists of taq Activation 95°C for 10mins, Denaturation: 94 for 30 sec, Annealing: 58. 8 °C for 1min, Extension: 72 °C for 20 seconds and Annealing/Extension: 72 °C for 7mins

PCR amplification is verified on a 1.5% agarose gel. PCR products are purified from the other PCR mixture components using the Stratagene Strataprep PCR Purification Kit. The purified SCTE PCR product is used as the template for restriction enzyme PCRRestriction Enzyme Site Flanked PCR.

The insect cell vector pMT/VS-His (Invitrogen, Cat #K4120-20) is chosen for cloning. This vector is available in 3 reading frames, A, B and C. Each reading frame facilitates cloning with expression of the C-terminal peptide. The correct reading frame is chosen with respect to the start codon of the SCTE DNA insert.

Restriction enzyme (RE) sites around the multiple cloning site of the vector, which are in frame with the SCTE start codon, are chosen. The SCTE DNA sequence is screened for the presence of restriction enzyme sites using the Webcutter program found on the NIH web site (www. nih. gojp/-jun/research/anal. html). Those restriction enzyme sites not found in the SCTE sequence, but found on the vector are potentially available for use.

The SCTE start codon is found to be in frame with the pMT/V5-HisC vector. Kpnl is chosen as the first restriction enzyme, at the start codon. Notl is chosen as the second primer, after the stop codon. Restriction enzyme sites are incorporated into the ***forward and reverse SCTEPCR primers*** in order to generate a SCTE PCR product flanked by Kpnl and Notl. These sites enable the sequence to be inserted into the vector:
*SCTE Forward Restriction Enzyme Primer (5' to 3)*
ATTAGTA-GGGTAG-ATGGCTACAGCAAGACCC
Random Sequence-Kpnl Restriction Enzyme Sequence-SCTE Start codon sequence
*SCTE Forward RE Primer (5' to 3)*
**ATTAGTA GGTACC ATG**GCTACAGCAAGACCC
• Random SequenceKpnl RE Seq SCTE Start codon sequence
*SCTE Reverse RE Primer (3' to 5)*
1) TCCAGGCCAACTC **CTGA GCGGCCGC ATATTA**
2) SCTE Stop codon sequence Notl RE Seq Random Sequence
*SCTE Reverse RE Primer (5' to* 3*) (compliment)*
TAATATGCGGC CGCTCAGGAG TTGGCCTGGA

SCTE PCR product from standard primer PCR is used as the template for Restriction Enzyme-primer PCR. PCR conditions are 2.5mM Mg²⁺ at any annealing temperature between 57°C and 67°C. The SCTE-Restriction Enzyme PCR product is digested with the corresponding restriction enzymes (Kpnl and Notl) according to the manufacturers recommended protocol (Promega). The digested SCTE-Restriction Enzyme PCR product is purified from the restriction enzyme mix by agarose gel electrophoresis and the DNA purified from the gel using the Qiagen QiaexII Agarose Gel Extraction Kit. The SCTE insert is now ready for ligation to the prepared vector.

### PMT/V5-HisC Vector Propagation and Restriction Enzyme Digestion

The pMT/V5-HisC vector is propagated in Invitrogen TOP 10 chemically modified E. Coli Cells (Invitrogen, Cat #C4040-10). The protocol is described in the TOP 10 information booklet: Add 1 µl of the vector to a vial of TOP 10 cells (which have been thawed on ice). Gently mix. Incubate for 5 to 30mins on ice. Heat-shock cells for 30 seconds at 42 °C, without shaking. Immediately transfer back to ice. Add 250 µl of room temperature SOC buffer. Cap the tube tightly and shake to tube horizontally at 37 °C for 1hour. Evenly spread 50µl of transformant mix onto a fresh agar plate containing ampicillin. Incubate overnight at 37 °C. Single colonies are picked up and used to inoculate ampicillin-LB, and the culture incubated overnight at 37 °C. Glycerol stocks are made of the cultures. The propagated pMT/V5-HisC vector is purified from the bacteria using the Qiagen DNA Mini-Prep Kit, according to the manufacturers recommended protocol.

Purified vector is digested with Kpnl and Notl, as for the SCTE-Restriction Enzyme PCR product. Digested vector is also purified from the restriction enzyme mix by agarose gel electrophoresis and the DNA purified from the gel using the Qiagen QiaExII Agarose Gel Extraction Kit. The vector is now ready for ligation to the prepared SCTE insert.

### pMTlV5-HisC and SCTE Vector Ligation

The SCTE is ligated to the pMT/V5-HisC vector using the enzyme T4-DNA Ligase (Gibco, Cat #; 15224-017). Ligation performed at a ratio of 5: 1, DNA insert: vector according to the following protocol: DNA Ligase (1U/, µl) 1 µl, 5 x Ligation Buffer 4µl, SCTE Insert (X µg/µl), pMT vector ((X µg/µl), Water (up to 20µl). The ligation reaction is incubated at 16 °C overnight (floating on icey water at 16 °C). The ligation reaction is assessed by 0.7% agarose gel (compared to unligated control samples). Competent E-Coli cells are transformed with ligated SCTE/Vector construct for propagation.

Propagation of SCTE/Vector Construct in E-Coli CellsTOP10 cells are transformed with the construct according to the manufacturers protocol, described above. The transformed cells are streaked onto ampicillin-agar plates and incubated overnight at 37°C. Single colonies are used to inoculate ampicillin-LB and the culture incubated overnight at 37 °C. Glycerol stocks have been made of the cultures. The construct is purified using the Qiagen DNA Mini-Prep Kit.

### Lipid Transfection of S2 Insect Cells

S2 Insect cells (drosophila, from Invitrogen, Cat #R690-07) are cultured as recommended by the manufacturer-in Drosophila Expression Medium (DES, Cat #;Q500-01) supplemented with foetal calf serum and penicillin-streptomycin. Transfection is performed using Cellfectin (Invitrogen, Cat #;10362-010), a liposome formulation, again according to the manufacturers protocol.

### Induction of SCTE Protein Expression/Formation of Stable Cell Lines

The pMT/V5-HisC vector has a metal-inducible (inducible with copper sulphate) promoter, metallothionein (MT), which enables high level expression of the target gene in S2 cells. Protein expression in the cells is induced with copper sulphate, added to a final concentration of 500µM. Cells are incubated for 1-4 days. Following induction, cells are harvested and stored until further analysis. Polyacrylamide gel electrophoresis (PAGE) analysis is used to determine whether recombinant protein expression has occurred by comparing the total protein of induced cells and non- induced cells.

### Formation of Stable Cell Lines

After demonstrating that the SCTE protein is expressed in S2 cells, expression is scaled up by creating stable cells lines. Cells produce blasticidin, which is a potent translational inhibitor in prokaryotic and eukaryotic systems. Resistance to blasticidin is conferred after co-transfection of the SCTE/pMT/V5-HisC construct and pCoBlast selection vector (Invitrogen, Cat #1K5150-01) into S2 cells. The protocol is found in the Drosophila expression system (DES) manual, which accompanies all DES reagents. Blasticidin (25µg/ml) is used to select stable transfectants.

### Scale-up of Protein Expression

Expression is scaled up for protein purification by using larger volumes/flasks. The Invitrogen manual enclosed with the S2 cells provides details of a protocol for doing this. Purification is performed by FPLC. Different fractions are collected and analysed for the presence of the recombinant protein.

### Example C2. Production of Recombinant SCCE

Stratum Corneum Chymotryptic Enzyme (SCCE) is cloned and expressed in an active form such that it has full enzymatic activity.

### Reverse Transcription-Polymerase Chain Reaction (RT-PCR)

PCR Primers, covering the open reading frame, are designed manually i.e. by sight using SCCE sequence (GB: NM005046). All primers are from Invitrogen.
**Forward** (5'TO 3'): CGG GCT CCA TGG CAA GAT C
**Reverse** (5'TO 3'): GCG TCC TCA CTC CTG TGC

Human epidermis total RNA reverse transcribed to cDNA with the Applied Biosystems Gold RNA PCR Kit. RT Cycle is as previously described for SCTE, using 2.0mM Mg²⁺, 40 cycles, PCR Cycle is as described for SCTE, except that annealing is done at 60oC for 1 min.

### Restriction Enzyme Site Flanked PCR

The correct reading frame and Restriction Enzyme sites around the multiple cloning site is chosen with respect to the start codon of the SCCE DNA insert. The SCCE DNA sequence is screened for the presence of restriction enzyme sites using the Webcutter program, and restriction enzyme sites chosen as described previously. The SCCE start codon is found to be in frame with *the pMT*/*V5-HisA* vector. EcoRl is chosen as the first restriction enzyme, at the start codon. Xho 1 is chosen as the second primer, after the stop codon.

Restriction enzyme sites are incorporated into *fhe **forward and reverse SCCE PCR primers*** in order to generate a SCCE PCR product flanked by EcoRI and Xhol. These sites enable the sequence to be inserted into the vector :-SCCE Forward Restriction Enzyme Primer (5' to 3')
GCC AGC-GAA TTC-ATGGCA AGA TCC CTT CTC
Random Sequence-EcoRl Restriction Enzyme Sequence-SCCE Start codon sequence
*SCCE Reverse Restriction Enzyme Primer (3' to 5)*
ATGAAAAAGCATCGCTAA-CTCGAG-AGCACT
SCCE Stop codon sequence-Xhol Restriction Enzyme Sequence-Random Sequence *SCCE Reverse Restriction Enzyme Primer (5' to* 3*) (compliment)*
AGTGCTCTCG AGTTAGCGAT GCTTTTTCAT

SCCE PCR product from standard primer PCR is used as the template for Restriction Enzyme-primer PCR. PCR conditions are 2.0mM Mg²⁺ at an annealing temperature of 62°C. The SCCE-Restriction Enzyme PCR product is digested with the corresponding Restriction Enzyme's (EcoR1 and XhoI)-according to the manufacturers recommended protocol (Promega). The digested SCCE-Restriction Enzyme PCR product is purified from the restriction enzyme mix by agarose gel electrophoresis and the DNA purified from the gel using the Qiagcn QiaexII Agarose Gel Extraction Kit. The SCCE insert is now ready for ligation to the prepared pMT/V5-HisA vector.

Expression is scaled up for protein purification by using larger volumes/flasks. The Invitrogen manual enclosed with the S2 cells provides details. Purification is performed by FPLC. Different fractions are collected and analysed for the presence of the recombinant protein. Further details for expression and purification of SCCE are disclosed in Hansson et al., 1994 (J. Biol. Chem. 269 (30), 19420-19426), which describes the purification of the protease enzyme SCCE.

### Example C3. Cleavage of Adhesion Proteins with Recombinant SCCE and SCTE

Recombinant ProSCCE is produced and purified as described above. Recombinant ProSCCE is activated with agarose-bound trypsin, as described in Brasttsand & Egelrud, 1999.

Proteins are extracted from human epidermis in the presence of a detergent (TENP-40 buffer extract) and incubated with active SCCE for 37C for 4 h. The reactions are stopped by boiling for 2 mins after Laemmli's sample buffer is added. The proteins are immunoblotted with antibodies against corneodesmosomal proteins.

### Cleavage of Adhesion Proteins by SCCE

We demonstrate with this Example that recombinant SCCE is able to cleave adhesion proteins, in particular, Corneodesmosin, Plakoglobin Desmoglein, Desmoplakin, Envoplakin and Desmocollin. Therefore, these adhesion proteins are substrates of this protease.

SCCE and Corneodesmosin: Corneodesmosin is found to be proteolysed by recombinant SCCE. The native form of corneodesmosin has a molecular weight of between 50 and 56 kDa. After 2h incubation the major forms of Corneodesmosin are 36 and 46-43 kDa.

### Cleavage of Adhesion Proteins by SCTE

We demonstrate with this Example that recombinant SCTE is able to cleave adhesion proteins, in particular, Comeodesmosin, Plakoglobin Desmoglein, Desmoplakin, Envoplakin and Desmocollin. Therefore, these adhesion proteins are substrates of this protease.

SCTE and Corneodesmosin: Comodesmosin is found to be proteolysed by recombinant SCTE. After 2h incubation the major forms of Corneodesmosin are 36 and 46-43 kDa

### Example C4. Proteolysis Profiles Materials and Methods

### Polyclonal Antibody Production

Antibodies to corneodesmosin, SCCE, envoplakin, desmoplakin, desmocollin 1 and SLPI are produced in rabbits by injection of synthetic peptides conjugated with keyhole limpet hemocyanin (KLH).

Peptides are coupled to a keyhole limpet hemocyanin (KLH) using standard procedures. The peptides are designed comprising the following amino acid sequences, derived from the relevant GenBank sequences as shown in Table C4.1 below:

**Table C4.1. Bold C residues are synthesised for coupling and do not exist in the native sequence.**

| Protein | Peptide Sequence | Name | GenBank Sequence |
|---|---|---|---|
| Corneodesmosin | FTKENPVKGSPGVC | SB2 | GB: AF030130 |
| SCCE | INDTMKKHR | SB1 | GB: XM009002 |
| SCCE | RRAQRIKASKS | SB4 | GB: XM_009002 |
| Envoplakin | SASPTVPRSLR | SB3 | GB: XM_008135 |
| Desmoplakin | SGKRDKSEEVQC | 642 | GB: XM_004463 |
| Desmocollin 1 | MENSLGPFPQC | 641 | GB: XM_004463 |
| SLPI | CGKSCVSPVKA | 644 | GB: X04502 |

Rabbits are injected with peptide coupled to KLH Rabbits at day 1. A brief protocol follows. Day 1: mix approximately 150µl (containing 300µg of conjugate) with an equal volume of Freunds Complete Adjuvant by passing several times through a 23G needle until an emulsion (which does not separate on standing) is formed. Inject sub-cutaneously into the rabbit using a 25G needle. Day 22, repeat the above but use Freunds Incomplete Adjuvant. Day 43, repeat the above exactly. Day 53, take first test bleed from ear vein. Boost and re-bleed as necessary.

### Monoclonal Antibodies

Monoclonal antibodies against adhesion protein antigens are obtained commercially from Biotechnic, Germany. These comprise anti-plakoglobin antibody directed against PG5. 1 (Plakoglobin (NM-021991)) and anti-desmoglein 1 (XM- 008810) antibody directed against Dsgl and Dsg2.

### Protein Extraction from the Epidermis

Proteins are extracted from biopsies in the form of "dog ears", which are triangular pieces of skin removed to produce a neat linear scar. This is not skin removed from the mastectomy specimen sent to the pathologists for diagnostics. All samples are from female patients attending clinics at the University of Sheffield undergoing mastectomy for breast carcinoma. Informed consent is obtained from the patients.

The epidermis is separated from the dermis by incubating a breast biopsy from breast surgery with trypsin solution A (Life Technology, France) for 18 h at 4 C. The epidermis is divided on two parts. One part is used for protein extraction and the other for RNA extraction (see below). The peeled epidermis is heated 5 mins in phosphate- buffuered saline at 56C (Simon et al, 1997). The epidermis is homogenized on ice on in equal volume of the following buffers (three times in each buffer): 40 mM Tris-HCl, pH 7.5,10 mM EDTA, 0.25 mM phenylmethylsulfonyl fluoride, and 2 µg/ml each of aprotinin, pepstatin A, and leupeptin (TE buffer), TE buffer containing 0.5% Nonidet P-40 (TE-Nonidet P-40 buffer).

The pellet is then divided into three parts that are extracted in one-third of the original volume of TE buffer containing various concentrations of urea (4, 6, and 8 M) (TEU buffers). After each extraction, the homogenates are centrifuged for 15 mins at 15,000 x g, and supernatants are kept at-30C until used inally, the pellet corresponding to the last extraction in 8 M urea is homogenized in 35 mM Tris-HCl, pH 6.8,8M urea, 50 mM dithiothreitol, 5% glycerol, 0.25 mM phenylmethylsulfonyl fluoride, and 2 g/ml each of aprotinin, pepstatin A, and leupeptin (TUDTT buffer), incubated at 95C for 30 mins, and centrifuged as described above (this method is originally described by Simon et al, 1997). Protein concentrations are measured using the Coomassie Plus protein assay (Pierre Chemical Co, Rockford, IL).

### Protein Extraction from Stratum Corneum

Adhesion proteins are extracted from the stratum corneum according to a method described by Guerrin et al, 1998. Briefly, a tape strip is applied to a biopsy from normal skin or lesional and non-lesional of a patient with psoriasis. The tape strips are incubated in acetone and the tissue is recovered by centrifugation (500 x g, 1min), washed in acetone, and air-dried. The powder is boiled for 10 mins in 62.5 mM Tris-HCl, pH 6.8, containing 2% SDS and 50 mM DTT, and the solution is centrifuged (10,000 x g, 10 mins).

Proteins are extracted from the stratum corneum of normal and psoriatic patient groups, as described above, and analysed by Western blots using specific antibodies against corneodesmosomal proteins. We show that the profile of epidermally extracted proteins differs between normal and diseased (psoriatic) individuals.

### Western blot

Proteins from the epidermal and stratum corneum biopsies (~1µg) are separated by 10% SDS-polyacrylamide gel electrophoresis (SDS-PAGE). After electrophoresis the proteins are electrotransferred to nitrocellulose membranes. Membranes are blocked overnight at 4°C in Blotto (3% milk powder, 2% BSA, 0.1% Tween 20 in TBS). The membranes are probed with the primary antibodies described below for 3 hours at room temperature with agitation. Mouse monoclonal antibodies directed against plakoglobin and desmoglein are obtained from Progen (Heidelberg, Germany) and used at a 5µg/ml concentration, diluted in Blotto. Rabbit polyclonal antibodies directed against peptides designed from protein sequences of desmocollin, desmoplakin, SLPI, SCCE, S protein and envoplakin (Antibody Resource Centre, Sheffield University, UK) are used at a 1:250 dilution. Membranes are washed for 3 x 5mins in Blotto and incubated in the presence of the secondary antibody, either anti- mouse or anti-rabbit IgG HRP obtained from Santa Cruz Biotechnology, Inc. (California, USA) at a 1: 1000 dilution for 1 hour at room temperature, with agitation. The membranes are washed in TBS-Tween 20 (0.1 %) for 3 x 5 mins and proteins detected using the ECL+Plus^{™} western blotting detection system (Amersham Pharmacia Biotech, Little Chalfont, UK),

### Example C7. Eczema Proteolysis Profiles of Proteins Extracted from the Stratum Corneum

Proteins are extracted from the stratum corneum of normal and eczematous patient groups, as described above, and analysed by Western blots using specific antibodies against corneodesmosomal proteins. We show that the profile of epidermally extracted proteins differs between normal and diseased (eczema) individuals.

### Corneodesmosome Proteolysis Profile

The increase in proteolysis of desmo/corneodesmosomal proteins in eczema skin is reflected in a significant increase in the quantity of immature forms of corneodesmosome proteins in eczematous skin compared to normal. Increased proteolysis causes the cells to stick together in the surface of the epidermis preventing cell shedding.

For example, the 36 and 46-43 kDa forms of corneodesmosin are the major forms in the stratum corneum in normal skin and the 52-56 kDa forms are very rare in the stratum corneum. There is an increase of corneodesmosin proteolysis in eczematous skin, so that the 36 and 46-43 kDa forms of corneodesmosin are dominant forms in the stratum corneum of eczema patients. This shows that there is an increase in proteolysis in eczematous skin.

We therefore disclose the diagnosis of a Group I disease or susceptibility to a Group I disease (preferably eczema or susceptibility to eczema) in an individual, by detecting any of the changes described above, preferably the presence of or a modulated level, preferably a higher level of one or more 36,46-43 kDa corneodesmosin polypeptides in an individual. We also disclose the diagnosis of a Group I disease or susceptibility to such a disease (preferably eczema or susceptibility to eczema) in an individual, by detecting the absence of or a modulated level, preferably a lower level of 52-56 kDa corneodesmosin polypeptides in an individual.

The diagnosis may also be achieved by assaying the relative abundance of the 36 kDa, 46-43 kDa and 52-56 kDa polypeptides.

Preferably, the relevant polypeptides are detected in an stratum corneum of an individual, whether in vivo or ex-vivo (e. g., in the form of a tape strip).

### EXAMPLES D: GENE REGULATION

### Example D1. Expression of Adhesion Protein, Protease and Protease Inhibitor Genes in Psoriasis Assayed Using Oligonucleotide Arrays

We used Affymetrix oligonucleotide arrays comprising desmosomal and corneodesmosomal adhesion proteins, proteases and protease inhibitor genes. These genes are listed in the Tables below in Examples D2 to D7.

Punch biopsies from involved and uninvolved skin of patients with psoriasis, acne vulgaris, ichtyosis, keratoses pilaris, atopic eczema, Crohn's disease, skin melanoma, squamous cell carcinima, basal cell carcinima, cutaneous lymphoma, skin cancer, malignancy of gastrointestinal tract, malignancy of the lung, are obtained from the skin. Biopsies are also obtained from unrelated control blood donors.

RNA is extracted from each sample and an average of 50, 20 and 15 µg are obtained from lesional, non-lesional and normal skins respectively. Approximately 10 µg is used to prepare biotinylated cRNA and 2 µg is used for hybridization to Affymetrix U95A arrays that contain probes for genes as listed in the Tables.

GENECHIP software is used to reflect the expression level of each gene by converting the image intensities to average difference into the expression level. The expression of genes in normal skin is used as reference and the results are shown in the Tables below.

### Example D5. Expression of Corneodesmosomal Genes in Eczema Assayed Using Oligonucleotide Arrays

The expression of corneodesmosomal genes is assayed as described in eczema patients, using eczema lesional skin ("involved") and eczema non-lesional skin ("uninvolved"). The expression level of each gene in disease (involved and uninvolved) is compared to its expression in normal skin.

Results are shown in Table D5.1 below. Key: ++: normally expressed , +++ : strongly expressed; ++++ : highly expressed, + down-regulation.

**Table D5. 1.**

| **Gene Name** | **Expression level** | | **GenBank accession number** |
|---|---|---|---|
| | **Involved** | **Uninvolved** | |
| S/comeodesmosin | + | + | GB: AF030130 |
| Desoplakin | + | + | GB: XM 004463 |
| Plakoglobin | + | + | GB: NM_002230; GB: NM 021991 |
| desmoglein 1 | + | + | GB: XM 008810 |
| desmocollin 1 | + | + | GB: MX 008687 |
| Envoplakin | + | + | GB: XM 008135; U72543 |
| plectin 1 | + | ++ | GB: NM000445 |
| S100A2 | + | ++ | GB: AI539439 ; M87068 |
| keratin 6A | ++ | ++ | GB: L42611 |
| keratin 17 | ++ | ++ | GB: Z19574 |
| S100A8 | + | ++ | GB: AI126134 |
| S 100A7 | + | +++ | GB: AA586894 |
| S100A9 | + | ++ | GB: W72424 |
| SPRR2A | + | ++ | GB: M21302 |
| SPRR1B | + | + | GB: M19888 |
| SPRK | + | + | GB: AI923984 |
| HCR | + | + | GB : BAA81890 |
| SEEK1 | + | + | GB: BAA88130 |
| SPR1 | + | + | GB: BAB63315 |
| STG | + | ++ | GB: BAA88132 |
| Involucrin | + | + | GB: NM 005547 |
| annexin A1/li ocortin | ++ | ++ | GB: X05908 |
| Trichohyalin | + | + | GB: NM 005547 |
| collagen, type VI, alpha 3 (COL6A3) | ++ | ++ | GB: NM_004369 |
| Loricrin | + | + | GB: XM 048902 |

Wc therefore provide the diagnosis of a Group 1 disease or susceptibility to a Group 1 disease (preferably eczema or susceptibility to eczema) in an individual, by detecting modulation of expression, preferably up-regulation of expression of a polypeptide or nucleic acid selected from the group consisting of keratin 6A (L42611); keratin 17 (Z19574); annexin A1/lipocortin (X05908); and collagen, type VI, alpha 3 (COL6A3) (NM_004369) in an individual.

Furthermore, we provide the diagnosis of a Group 1 disease or susceptibility to a Group 1 disease (preferably eczema or susceptibility to eczema) in an individual, by detecting modulation of expression, preferably down-regulation of expression of a polypeptide or nucleic acid selected from the group consisting of S/comeodesmosin (AF030130); desoplakin (XM-004463); plakoglobin (NM_002230; (NM_021991); desmoglein 1 (XM_008810); desmocollin 1 (MX_008687); envoplakin 9XM_08135; U72543); plectin 1 (NM000445); S100A2 (AI539439; M87068); S100A8 (A1126134); S100A7 (AA586894); S100A9); GB: W72424); SPRR2A); GB: M21302); SPRR1B (M19888); SPRK (AI923984); HCR (BAA81890); SEEK1 (BAA88130); SPR1 (BAB63315); STG (BAA88132); involucrin (NM_005547); trichohyalin (NM_005547); and loricrin (XM_048902).

### REFERENCES

Allen MH, Veal C, Faassen A, Powis SH, Vaughan RW, Trembath RC, Barker JNWN. (1999). A non-HLA gene within the MHC in psoriasis. Lancet 353(9164); 1589-1590.
Brattsand M, Egelrud T. (1999). Protein purification, molecular cloning, and expression of a human stratum corneum trypsin-like serine protease with possible function in desquamation. J Biol Chem. 274 (42): 30033-40.
Chapman and Walsh (1990). Desmosomes, corneosomes and desquamation. An ultrastructural study of adult pig epidermis. Arch Dermatol Res 282(5); 304-310.
Cork, M. J. (1997) The importance of skin barrier function. J. Derm Treatment. 8, S7-S13.
Egelrud T. (1993). Purification and preliminary characterization of stratum corneum chymotryptic enzyme: a proteinase that may be involved in dequamation. J Invest dermatol 101, 200-204.
Egelrud, T. (2000) Desquamation in the stratum corneum. Acta. Derm. Venerol. Supp. 208, 44-45.
Ekholm IE, Brattsand M, Eglrud T. (2000). Stratum corneum tryptic enzyme in normal epidermis: a missing link in the desquamation process. J Invest Dermatol, 114, 56-63.
Ekholm, E. and Egelrud, T. (1998) The expression of stratum corneum chymotrytic enzyme in human anagen hair follicles: further evidence for its involvement in desquamation-like processes. Brit. J Derm. 139, 585-590.
Elias, P. M. (1983) Epidermal lipids, barrier function and desquamation. J. Invest. Dermatol. 80, (6) 44-49.
Guerrin M, Vincent C, Simon M, Tazi Ahnini R, Fort M, Serre G. (2001). Identification of six novel polymorphisms in the human corneodesmosin gene. Tissue Antigens. 57 (1) : 32-8.
Guerrin, M., Simon, M., Montézin, M., Haftek, M., Vincent, C. and Serre, G. (1998) Expression cloning of human corneodesmosin proves its identity with product of the S gene and allows improved characterisation of its processing during keratinocyte differentiation. J Biol. Chem., 273 22640-22647.
Hansson L, Stromqvist M, Backman A, Wallbrandt P, Carlstein A, Egelrud T. (1994). Cloning, expression, and characterization of stratum corneum chymotryptic enzyme. A skin-specific human serine proteinase. J Biol Chem. 269 (30): 19420-6.
Ishihara M, Yamaguata N, Ohno S, Naruse T, Ando A, Kawata H, Ozawa A, Ohkido M, Mizuki N, Shiina T, Ando H, Inoko H. (1996) Genetic polymorphisms in the keratin-like S gene within the human major histocompatibility complex and association on the susceptibility to psoriasis vulgaris. Tissue Antigens, 48: 182- 186.
Jenisch S, Koch S, Henseler T, Nair RP, Elder JT, Watts CE, Westphal E, Voorhees JJ, Christophers E, Kronke M. (1999). Corneodesmosin gene polymorphism demonstrates strong linkage disequilibrium with HLA and association with psoriasis. Tissue Antigens, 54: 439-449.
Landmann, L. (1988) The epidermal permeability barrier. Anat. Embryo. (Berl.). 172, 1-13.
Menton and Eisen (1971). Structure and organisation of mammalian stratum corneum. J Utrastruct. Res 35: 247-264
North AJ, Bardsley WG, Hyam J, Bornslaeger EA, Cordingley HC, Trinnaman B, Halzfeld M, Green KJ, Magee AI, Garrod DR. Molecular map of the desmosomal plaque. Journal of Cell Science 112,4325-4336 (1999).
Simon, M., Montézin, M., Guerrin, M., Durieux, J. J. and Serre, G. (1997) Characterisation and purification of human corneodesmosin, an epidermal basic glycoprotein associated with corneocyte-specific modified desmosomes. J: Biol.Chem., 272,31770-31776.
Tazi Ahnini R, Camp NJ, Mee JB, Duff GW, Cork M, di Giovine FS (1999a). Genetic Association between the corneodesmosin (MHC) S Gene and Susceptibility to Psoriasis. Hum. Mol. Genet. 8 (6): 1135-1140.
Tazi Ahnini R, di Giovine FS, Cox A, Keohane and Cork MJ. The corneodesmosin (MHC S) gene in Guttate psoriasis. Lancet 1999 14; 354: 597 .

## Claims

1. A method of providing indications useful in the diagnosis of a disease, or susceptibility to a disease associated with decreased cell-cell adhesion between epithelial cells
wherein the disease is selected from the group consisting of atopic eczema, sebarrhoeic eczema, irritant contact dermatitis, allergic contact dermatitis, lung atopic asthma, post viral asthma, bronchial hyper-reactivity, chronic obstruction pulmonary disease, Crohn's disease, ulcerative colitis, coeliac disease, peptic ulceration, impetigo, viral warts, Molluscum Contagiosum, bacterial meningitis, viral meningitis, peptic ulceration associated with penetration of *Helicobacteria pylori*, skin melanoma, squamous cell carcinoma, basal cell carcinoma, cutaneous lymphoma, a skin cancer, a malignancy of gastrointestinal tract and a malignancy of the lung,
the method comprising detection of a mutation in corneodesmosin selected from detecting any one or more of:
(a) the presence of a T at position +1243 of a corneodesmosin nucleic acid;
(b) the absence of a H*ph*1 restriction enzyme site at position of +1243 of a corneodesmosin nucleic acid;
(c) the presence of a leucine (L) residue at position 394 of a corneodesmosin polypeptide (L20815.1);
(d) a mutation in a corneodesmosin nucleic acid which leads to (c);
(e) the following nucleotides or any one or more of the following amino acids at the relevant positions of a corneodesmosin nucleic acid or polypeptide:
| Nucleotide Position | 442 | 468 | 619 | 1215 | 1236 | 1243 | 1515 | 1593 |
|---|---|---|---|---|---|---|---|---|
| Nucleic acid(s) | A | AGT | T | A | T | T | G | T |
| Residue Position (1) | 127 | 137 | 186 | 385 | 392 | 394 | 485 | 511 |
| Residue Position (2) | 143 | 153 | 202 | 401 | 408 | 410 | 501 | 527 |
| Residue | D | S/- | F | S | S | L | D | D/N |
in which "Residue Position (1)" refers to the numbering of the sequence with accession number L2081.1, and "Residue Position (2)" refers to the numbering of the sequence accession number AF030130.1;
(f) the presence of a T at position 180 of a corneodesmosin nucleic acid;
(g) the presence of an F at position 40 of a corneodesmosin polypeptide having accession number L20815.1;
(h) the presence of an F at position 56 of a corneodesmosin polypeptide having accession number AF030130.1;
(i) a mutation in a corneodesmosin nucleic acid which leads to (g) or (h);
(j) the presence of a T at position 619 of a corneodesmosin nucleic acid;
(k) the presence of an F at position 186 of a corneodesmosin polypeptide; and
(l) a mutation in a corneodesmosin nucleic acid which leads to (k),
in a sample of cells or bodily fluid obtained from a subject.

2. A method according to claim 1 wherein the decreased adhesion is between corneocytes.

3. A method according to claim 1 or claim 2 wherein the disease is selected from the group consisting of: atopic eczema, sebarrhoeic eczema, irritant contact dermatitis, allergic contact dermatitis and impetigo.

4. A method according to any one of the preceding claims comprising detecting a CD5 corneodesmosin allele or a CD6 corneodesmosin allele in an individual.

5. A method of providing indications useful in the diagnosis of a disease or susceptibility to a disease associated with decreased cell-cell adhesion between epithelial cells in an individual,
wherein the disease is selected from the group consisting of: atopic eczema, sebarrhoeic eczema, irritant contact dermatitis, allergic contact dermatitis and impetigo,
the method comprising detecting the presence, absence or a modulated level of corneodesmosin or a fragment thereof, in a sample of cells or bodily fluid obtained from a subject.

6. A method according to claim 5, in which the method comprises detecting any one or more of: (a) relative abundance of the 36 kDa, 46-43 kDa and 52-56 kDa corneodesmosin polypeptides; (b) the presence of or an elevated level of one or more 36, 46 -43 kDa corneodesmosin polypeptides; (c) the absence of or a modulated level, preferably a lower level of 52-56 kDa corneodesmosin polypeptides in an individual.

7. A method according to any one of the preceding claims, in which a polypeptide or fragment is detected in an epidermis of an individual, preferably *ex-vivo* in the form of a skin biopsy, or in the stratum corneum of an individual, preferably in the form of a tape strip.

8. A method according to any one of the preceding claims which comprises detecting down-regulation of expression of an adhesion protein polypeptide or nucleic acid which is S/corneodesmosin (AF030130.1).

## Patentansprüche

1. Verfahren zum Bereitstellen von Indikationen, die bei der Diagnose einer Krankheit oder einer Anfälligkeit für eine Krankheit geeignet sind, die mit einer verminderten Zell-Zell-Adhäsion zwischen Epithelzellen zusammenhängt,
wobei die Krankheit aus der Gruppe, bestehend aus atopischem Ekzem, seborrhoischem Ekzem, nicht-allergischer Kontaktdermatitis, allergischer Kontaktdermatitis, konstitutionsallergischem Lungenasthma, postviralem Asthma, bronchialer Hyperreaktivität, chronisch-obstruktiver Lungenerkrankung, Morbus Crohn, Colitis ulcerosa, Zöliakie, peptischem Ulkus, Impetigo, virusbedingten Warzen, Dellwarze, bakterieller Meningitis, viraler Meningitis, peptischem Ulkus im Zusammenhang mit dem Eindringen von *Helicobacteria pylori*, Hautmelanom, Plattenepithelkarzinom, Basalzellkarzinom, kutanem Lymphom, einem Hautkrebs, einer bösartigen Erkrankung des Gastrointestinaltrakts und einer bösartigen Erkrankung der Lunge, ausgewählt ist,
wobei das Verfahren das Nachweisen einer Mutation in Corneodesmosin, ausgewählt aus dem Nachweisen von einem oder mehreren von:
(a) dem Vorliegen eines T an der Position +1243 einer Corneodesmosin-Nukleinsäure,
(b) dem Fehlen einer H*ph*1-Restriktionsenzymstelle an der Position +1243 einer Corneodesmosin-Nukleinsäure,
(c) dem Vorliegen eines Leucin (L)-Rests an der Position 394 eines Corneodesmosin-Polypeptids (L20815.1),
(d) einer Mutation in einer Corneodesmosin-Nukleinsäure, die zu (c) führt,
(e) den folgenden Nukleotiden oder einer oder mehreren der folgenden Aminosäuren an den relevanten Positionen einer Corneodesmosin-Nukleinsäure oder eines Corneodesmosin-Polypeptids:
| Nukleotidposition | 442 | 468 | 619 | 1215 | 1236 | 1243 | 1515 | 1593 |
|---|---|---|---|---|---|---|---|---|
| Nukleinsäure(n) | A | AGT | T | A | T | T | G | T |
| Rest-Position (1) | 127 | 137 | 186 | 385 | 392 | 394 | 485 | 511 |
| Rest-Position (2) | 143 | 153 | 202 | 401 | 408 | 410 | 501 | 527 |
| Rest | D | S/- | F | S | S | L | D | D/N |
wobei sich "Rest-Position (1)" auf die Nummerierung der Sequenz mit der Zugangsnummer L20815.1 bezieht und sich "Rest-Position (2)" auf die Nummerierung der Sequenz mit der Zugangsnummer AF030130.1 bezieht,
(f) dem Vorliegen eines T an der Position 180 einer Corneodesmosh-Nukleinsäure,
(g) dem Vorliegen eines F an der Position 40 eines Corneodesmosin-Polypeptids mit der Zugangsnummer L20815.1,
(h) dem Vorlagen eines F an der Position 56 eines Corneodesmosin-Polypeptids mit der Zugangsnummer AF030130.1,
(i) einer Mutation in einer Corneodesmosin-Nukleinsäure, die zu (g) oder (h) führt,
(j) dem Vorliegen eines T an der Position 619 einer Corneodesmosh-Nukleinsäure,
(k) dem Vorliegen eines F an der Position 186 eines Corneodesmosin-Polypeptids und
(l) einer Mutation in einer Corneodesmosin-Nukleinsäure, die zu (k) führt,
in einer Probe von Zellen oder Körperfluid, die von einem Lebewesen erhalten worden ist, umfasst.

2. Verfahren nach Anspruch 1, bei dem die verminderte Haftung zwischen Corneozyten vorliegt.

3. Verfahren nach Anspruch 1 oder Anspruch 2, bei dem die Krankheit aus der Gruppe, bestehend aus atopischem Ekzem, seborrhoischem Ekzem, nicht-allergischer Kontaktdermatitis, allergischer Kontaktdermatitis und Impetigo, ausgewählt ist.

4. Verfahren nach einem der vorhergehenden Ansprüche, umfassend das Nachweisen eines CD5-Corneodesmosin-Allels oder eines CD6-Corneodesmosin-Allels in einem Lebewesen.

5. Verfahren zum Bereitstellen von Indikationen, die bei der Diagnose einer Krankheit oder einer Anfälligkeit für eine Krankheit geeignet sind, die mit einer verminderten Zell-Zell-Adhäsion zwischen Epithelzellen in einem Lebewesen zusammenhängt,
wobei die Krankheit aus der Gruppe, bestehend aus atopischem Ekzem, seborrhoischem Ekzem, nicht-allergischer Kontaktdermatitis, allergischer Kontaktdermatitis und Impetigo, ausgewählt ist,
wobei das Verfahren das Nachweisen des Vorliegens, des Fehlens oder einer veränderten Konzentration von Corneodesmosin oder eines Fragments davon in einer Probe von Zellen oder Körperfluid, die von einem Lebewesen erhalten worden ist, umfasst.

6. Verfahren nach Anspruch 5, wobei das Verfahren das Nachweisen von einem oder mehreren von: (a) der relativen Menge der 36 kDa-, 46-43 kDa- und 52-56 kDa-Corneodesmosin-Polypeptide, (b) dem Vorliegen oder einer erhöhten Konzentration von einem oder mehreren 36-, 46-43-kDa-Corneodesmosin-Polypeptid(en), (c) dem Fehlen oder einer veränderten Konzentration, vorzugsweise einer niedrigeren Konzentration, von 52-56-kDa-Corneodesmosin-Polypeptiden in einem Lebewesen umfasst.

7. Verfahren nach einem der vorhergehenden Ansprüche, bei dem ein Polypeptid oder -fragment in einer Epidermis eines Lebewesens, vorzugsweise *ex-vivo* in der Form einer Hautbiopsie, oder im Stratum corneum eines Lebewesens, vorzugsweise in der Form eines Pflasterstreifens, nachgewiesen wird.

8. Verfahren nach einem der vorhergehenden Ansprüche, welches das Nachweisen einer Herabregulation der Expression eines Adhäsionsproteinpolypeptids oder einer Adhäsionsproteinnukleinsäure, wobei es sich um S/Corneodesmosin (AF030130.1) handelt, umfasst.

## Revendications

1. Procédé pour fournir des indications utiles dans le diagnostic d'une maladie, ou d'une prédisposition à une maladie associée à une diminution de l'adhésion de cellule à cellule entre des cellules épithéliales,
dans lequel la maladie est choisie dans l'ensemble constitué par l'eczéma atopique, l'eczéma séborrhéique, la dermatite de contact irritante, la dermatite de contact allergique, l'asthme pulmonaire atopique, l'asthme post-viral, l'hyperréactivité bronchique, la broncho-pneumopathie chronique obstructive, la maladie de Crohn, la recto-colite hémorragique, la maladie coeliaque, l'ulcère gastroduodénal, l'impétigo, les verrues virales, molluscum contagiosum, la méningite bactérienne, la méningite virale, l'ulcère gastroduodénal associé à une pénétration d'*Helicobacteria pylori*, le mélanome cutané, le carcinome à cellules squameuses, le carcinome à cellules basales, le lymphome cutané, un cancer de la peau, une malignité des voies gastro-intestinales et une malignité pulmonaire,
lequel procédé comprend la détection d'une mutation dans la cornéodesmosine, choisie parmi la détection d'un ou plusieurs quelconques parmi :
(a) la présence d'un T en position +1243 d'un acide nucléique de cornéodesmosine ;
(b) l'absence d'un site d'enzyme de restriction H*ph*1 en position +1243 d'un acide nucléique de cornéodesmosine ;
(c) la présence d'un résidu de leucine (L) en position 394 d'un polypeptide de cornéodesmosine (L20815.1) ;
(d) une mutation dans un acide nucléique de cornéodesmosine conduisant à (c) ;
(e) les nucléotides suivants ou un ou plusieurs quelconques des acides aminés suivants aux positions pertinentes d'un acide nucléique ou polypeptide de cornéodesmosine :
| Position de nucléotide | 442 | 468 | 619 | 1215 | 1236 | 1243 | 1515 | 1593 |
|---|---|---|---|---|---|---|---|---|
| Acide(s) nucléique(s) | A | AGT | T | A | T | T | G | T |
| Position de résidu (1) | 127 | 137 | 186 | 385 | 392 | 394 | 485 | 511 |
| Position de résidu (2) | 143 | 153 | 202 | 401 | 408 | 410 | 501 | 527 |
| Résidu | D | S/- | F | S | S | L | D | D/N |
où "Position de résidu (1)" se réfère à la numérotation de la séquence ayant le numéro d'accès L20815.1 et "Position de résidu (2)" se réfère à la numérotation de la séquence ayant le numéro d'accès AF030130.1 ;
(f) la présence d'un T en position 180 d'un acide nucléique de cornéodesmosine ;
(g) la présence d'un F en position 40 d'un polypeptide de cornéodesmosine ayant le numéro d'accès L20815.1 ;
(h) la présence d'un F en position 56 d'un polypeptide de cornéodesmosine ayant le numéro d'accès AF030130.1 ;
(i) une mutation dans un acide nucléique de cornéodesmosine conduisant à (g) ou (h) ;
(j) la présence d'un T en position 619 d'un acide nucléique de cornéodesmosine ;
(k) la présence d'un F en position 186 d'un polypeptide de cornéodesmosine ; et
(l) une mutation dans un acide nucléique de cornéodesmosine conduisant à (k),
dans un échantillon de cellules ou de fluide corporel obtenu à partir d'un sujet.

2. Procédé selon la revendication 1, dans lequel l'adhésion réduite est celle entre des cornéocytes.

3. Procédé selon la revendication 1 ou la revendication 2, dans lequel la maladie est choisie dans le groupe constitué par l'eczéma atopique, l'eczéma séborrhéique, la dermatite de contact irritante, la dermatite de contact allergique et l'impétigo.

4. Procédé selon l'une quelconque des revendications précédentes, comprenant la détection d'un allèle de cornéodesmosine CD5 ou d'un allèle de cornéodesmosine CD6 chez un individu.

5. Procédé pour fournir des indications utiles dans le diagnostic d'une maladie, ou d'une prédisposition à une maladie associée à une diminution de l'adhésion de cellule à cellule entre des cellules épithéliales chez un individu,
dans lequel la maladie est choisie dans l'ensemble constitué par l'eczéma atopique, l'eczéma séborrhéique, la dermatite de contact irritante, la dermatite de contact allergique, et l'impétigo,
le procédé comprenant la détection de la présence, de l'absence ou d'un niveau modulé de cornéodesmosine ou d'un fragment de celle-ci, dans un échantillon de cellules ou de fluide corporel obtenu à partir d'un sujet.

6. Procédé selon la revendication 5, lequel procédé comprend la détection d'un ou plusieurs quelconques parmi : une abondance relative des polypeptides de cornéodesmosine de 36 kDa, 46-43 kDa et 52-56 kDa ; (b) la présence ou un niveau élevé d'un ou plusieurs polypeptides de cornéodesmosine de 36, 46-43 kDa ; (c) l'absence ou un niveau modulé, de préférence un niveau réduit de polypeptides de cornéodesmosine de 52-56 kDa chez un individu.

7. Procédé selon l'une quelconque des revendications précédentes, dans lequel un polypeptide ou fragment est détecté dans l'épiderme d'un individu, de préférence *ex vivo* sous la forme d'une biopsie cutanée, ou dans la couche cornée d'un individu, de préférence sous la forme d'une bandelette.

8. Procédé selon l'une quelconque des revendications précédentes, qui comprend la détection d'une régulation à la baisse de l'expression d'un polypeptide ou acide nucléique de protéine d'adhésion qui est la S/cornéodesmosine (AF030130.1).
